# EUROPEAN PATENT APPLICATION

(11) **EP 4 729 582 A1**
(43) Date of publication of application: **22.04.2026**
(21) Application number: 24823477.5
(22) Date of filing: 14.06.2024
(51) Int. Cl.: C08L 101/14, C08L 39/06, C08L 65/00, C08L 79/00, C08L 79/04, C09K 23/00, H01B 1/12

(54) **WATER SOLUBLE COMPOSITION, METHOD FOR PRODUCING SAME, CONDUCTIVE COMPOSITION, AND METHOD FOR PRODUCING SAME**

(30) Priority: 14.06.2023 JP 2023097824
(71) Applicant: Mitsubishi Chemical Corporation, Tokyo 100-8251 (JP)
(72) Inventor: MORI, Takahiro, Tokyo 100-8251 (JP); CHATANI, Shunsuke, Tokyo 100-8251 (JP); IRIE, Yoshiko, Tokyo 100-8251 (JP); TAKAHASHI, Toshiya, Tokyo 100-8251 (JP); YANAGI, Mamiko, Tokyo 100-8251 (JP)
(74) Representative: Hoffmann Eitle
(86) International application number: PCT/JP2024/021737
(87) International publication number: WO 2024/257864

(57) **Abstract**

An object of the present invention is to provide a conductive composition having favorable coating properties and being less likely to generate foreign matter upon forming a coating film; a method for producing the same; a water soluble composition which is used in the conductive composition; and a method for producing the same. A water soluble composition including a water soluble polymer, in which the water soluble composition satisfies the following Conditions (A1) and (A2). In addition, a method for producing the water soluble composition, the method including a reprecipitation step of mixing a good solvent solution including the water soluble composition and a good solvent for the water soluble composition with a poor solvent for the water soluble composition, the poor solvent including an ether.
(A1) The water soluble polymer includes a nitrogen-containing functional group.
(A2) A 1.0% by mass aqueous solution of the water soluble composition has a surface tension at 25°C of 34.0 to 50.0.

## Description

### TECHNICAL FIELD

The present invention relates to a water soluble composition, a method for producing the same, a conductive composition, and a method for producing the same.

Priority is claimed on Japanese Patent Application No. 2023-097824, filed on June 14, 2023, the content of which is incorporated herein by reference.

### BACKGROUND ART

Pattern forming techniques using charged particle beams such as electron beams and ion beams are expected to be the next-generation techniques for optical lithography. When using charged particle beams, it is important to improve the sensitivity of a resist layer to improve productivity.

Accordingly, the use of highly sensitive chemical amplification type resist layers has become mainstream, in which an acid is generated in an exposed part or a part irradiated with charged particle beams and then a cross-linking reaction or a decomposition reaction is advanced by a heat treatment known as a post-exposure bake (PEB) treatment.

In addition, in recent years, the movement toward miniaturization of semiconductor devices has also led to a demand for resist shape management on the order of several nanometers.

Here, in pattern forming methods using charged particle beams, in particular, when the substrate has an insulating property, there is a problem in that the electric field generated by the charging (charge-up) of the substrate causes the trajectory of the charged particle beam to bend, making it difficult to obtain a desired pattern.

As a means for solving this problem, a technique in which a conductive composition including a conductive polymer is applied onto a surface of a resist layer to form a coating film and the surface of the resist layer is covered with the coating film to impart an antistatic function to the resist layer is already known to be effective.

In general, a surfactant is added to the conductive composition for the purpose of improving the coating properties.

For example, Patent Document 1 discloses, as a conductive composition having excellent coating properties and the like, a conductive composition including, in addition to a conductive polymer, a water soluble polymer having a nitrogen-containing functional group and a terminal hydrophobic group.

### Citation List

### Patent Document

Patent Document 1: Japanese Unexamined Patent Application, First Publication No. 2002-226721

### SUMMARY OF INVENTION

### Technical Problem

However, when the conductive composition includes a component that causes a defect (defect factor component), the component may aggregate during film formation and appear as foreign matter on the coating film. In a coating film in which foreign matter is generated, a problem such as disconnection of a line occurs after drawing with electron beams.

In the conductive composition described in Patent Document 1, there is still room for improvement in reduction of the defect factor component.

An object of the present invention is to provide a conductive composition having favorable coating properties and being less likely to generate foreign matter upon forming a coating film; a method for producing the same; a water soluble composition which is used in the conductive composition; and a method for producing the same.

### [Solution to Problem]

The present invention has the following aspects.
[1] A water soluble composition including:
   a water soluble polymer,
   wherein the water soluble composition satisfies the following Conditions (A1) and (A2),
   (A1) the water soluble polymer includes a nitrogen-containing functional group, and
   (A2) a 1.0% by mass aqueous solution of the water soluble composition has a surface tension at 25°C of 34.0 to 50.0.
[2] The water soluble composition according to [1],
   wherein the water soluble composition further satisfies the following Condition (A3),
   (A3) the water soluble composition includes more than 0 ppm by mass and 1.3 ppm by mass or less of a component quantified by the following measurement method,
      <Measurement Method>
   a sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition is dissolved in a mixed solvent of water and methanol at a volume ratio (water/methanol) of 2/8 to give a concentration of 0.2% by mass, such that the volume ratio (solution (I)/solution (II)) is 1/10, and
   after injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan.
[3] The water soluble composition according to [1] or [2],
   wherein the water soluble composition further satisfies the following Condition (A4),
   (A4) the water soluble composition includes more than 0 ppm by mass and less than 1.0 ppm by mass of a component quantified by the following measurement method,
      <Measurement Method>
   a sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition is dissolved in a mixed solvent of water and methanol at a volume ratio (water/methanol) of 2/8 to give a concentration of 0.2% by mass, such that the volume ratio (solution (I)/solution (II)) is 1/10, and
   after injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan.
[4] A water soluble composition including:
   a water soluble polymer,
   wherein the water soluble composition satisfies the following Conditions (B1) to (B3),
   (B1) the water soluble polymer includes a nitrogen-containing functional group,
   (B2) a 1.0% by mass aqueous solution of the water soluble composition has a surface tension at 25°C of 10.0 to 50.0, and
   (B3) the water soluble composition includes more than 0 ppm by mass and 1.3 ppm by mass or less of a component quantified by the following measurement method,
      <Measurement Method>
   a sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition is dissolved in a mixed solvent of water and methanol at a volume ratio (water/methanol) of 2/8 to give a concentration of 0.2% by mass, such that the volume ratio (solution (I)/solution (II)) is 1/10, and
   after injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan.
[5] The water soluble composition according to [4],
   wherein the water soluble composition further satisfies the following Condition (B4),
   (B4) the water soluble composition includes more than 0 ppm by mass and less than 1.0 ppm by mass of a component quantified by the following measurement method,
      <Measurement Method>
   a sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition is dissolved in a mixed solvent of water and methanol at a volume ratio (water/methanol) of 2/8 to give a concentration of 0.2% by mass, such that the volume ratio (solution (I)/solution (II)) is 1/10, and
   after injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan.
[6] The water soluble composition according to any one of [4] or [5],
   wherein the water soluble polymer has a mass-average molecular weight of 600 to 20,000.
[7] The water soluble composition according to any one of [1] to [6],
   wherein the water soluble polymer has a nitrogen-containing functional group.
[8] The water soluble composition according to any one of [1] to [7],
   wherein the water soluble polymer includes a pyrrolidone structure.
[9] A conductive composition including:
   the water soluble composition according to any one of [1] to [8]; and
   a conductive polymer.
[10] The conductive composition according to [9],
   wherein the conductive polymer includes a conductive polymer having at least one unit selected from units represented by General Formulae (1) to (4).

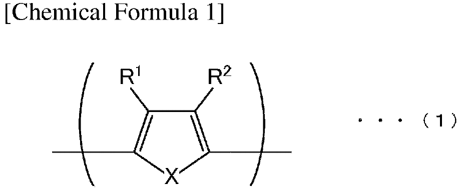

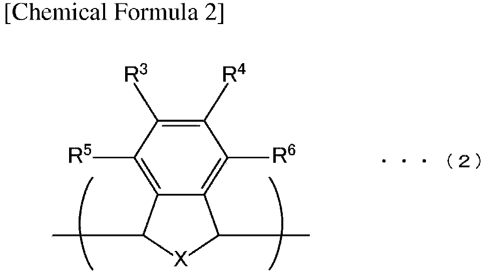

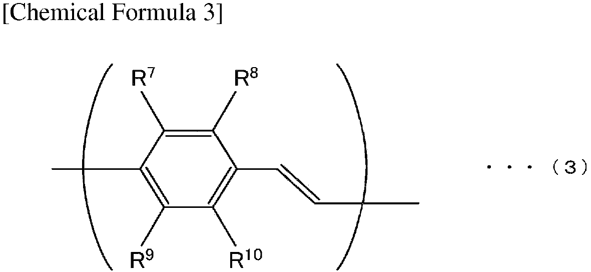

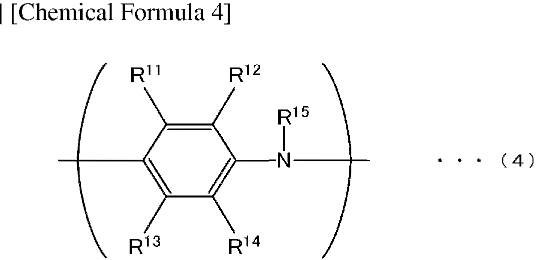

In General Formulae (1) to (4), X represents a sulfur atom or a nitrogen atom, and R¹ to R¹⁵ each independently represent a hydrogen atom, a linear or branched alkyl group having 1 to 24 carbon atoms, a linear or branched alkoxy group having 1 to 24 carbon atoms, an acidic group or a salt of the acidic group, a hydroxy group, a nitro group, a halogen atom, -N(R¹⁶)₂, -NHCOR¹⁶, -SR¹⁶, -OCOR¹⁶, -COOR¹⁶, -COR¹⁶, -CHO, or -CN. R¹⁶ represents an alkyl group having 1 to 24 carbon atoms, an aryl group having 6 to 24 carbon atoms, or an aralkyl group having 7 to 24 carbon atoms.

Provided that at least one of R¹ and R² in General Formula (1), at least one of R³ to R⁶ in General Formula (2), at least one of R⁷ to R¹⁰ in General Formula (3), and at least one of R¹¹ to R¹⁵ in General Formula (4) are each an acidic group or a salt of the acidic group.
[11] The conductive composition according to [9] or [10],
   wherein the conductive polymer includes a conductive polymer having a unit represented by General Formula (4). 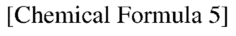 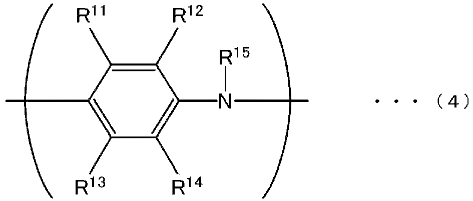 In General Formula (4), R¹¹ to R¹⁵ each independently represent a hydrogen atom, a linear or branched alkyl group having 1 to 24 carbon atoms, a linear or branched alkoxy group having 1 to 24 carbon atoms, an acidic group or a salt of the acidic group, a hydroxy group, a nitro group, a halogen atom, -N(R¹⁶)₂, -NHCOR¹⁶, -SR¹⁶, -OCOR¹⁶, - COOR¹⁶, -COR¹⁶, -CHO, or -CN, and R¹⁶ represents an alkyl group having 1 to 24 carbon atoms, an aryl group having 6 to 24 carbon atoms, or an aralkyl group having 7 to 24 carbon atoms.
[12] The conductive composition according to any one of [9] to [11], further including:
   a basic compound.
[13] The conductive composition according to [12],
   wherein the basic compound includes a quaternary ammonium compound.
[14] A method for producing the water soluble composition according to any one of [1] to [8], the method including:
   a reprecipitation step of mixing a good solvent solution including the water soluble polymer and a good solvent for the water soluble polymer with a poor solvent for the water soluble polymer,
   wherein the poor solvent includes an ether.
[15] The method for producing the water soluble composition according to [14], the method further including:
   a synthesis step of the water soluble polymer,
   wherein the reprecipitation step is performed after the synthesis step, and
   a reaction solution in the synthesis step is used as the good solvent solution in the reprecipitation step.
[16] The method for producing the water soluble composition according to [14] or [15],
   wherein a mixed amount of the poor solvent is 100 parts by mass or more with respect to 100 parts by mass of the good solvent solution.
[17] The method for producing the water soluble composition according to any one of [14] to [16],
   wherein the poor solvent further includes an alkane.
[18] The method for producing the water soluble composition according to [17],
   wherein a mass ratio of the alkane to the ether (alkane/ether) is 0.5 to 2.0.
[19] A method for producing a conductive composition, the method including:
   a step of producing a water soluble composition by the method for producing the water soluble composition according to any one of [14] to [18]; and
   a step of mixing the water soluble composition obtained in the step of producing the water soluble composition with a conductive polymer.

### Advantageous Effects of Invention

According to the present invention, it is possible to provide a conductive composition having favorable coating properties and being less likely to generate foreign matter upon forming a coating film; a method for producing the same; a water soluble composition which is used in the conductive composition; and a method for producing the same.

### DESCRIPTION OF EMBODIMENTS

The present invention will be described in detail below. The following embodiments are merely examples for describing the present invention and the present invention is not intended to be limited only to these embodiments. The present invention can be carried out in various aspects as long as the various aspects maintain the gist of the invention.

Furthermore, in the present invention, the "conductive" means having a surface resistivity of 1 x 10¹¹ Ω/□ or less. The surface resistivity is a surface resistance value per unit area (1 cm²) of a test material. The surface resistance value is determined from a potential difference between electrodes when a constant current flows therethrough.

In the present specification, the "solubility" means uniform dissolution of 0.1 g or more in 10 g (at a liquid temperature of 25°C) of one or more solvents including simple water, water including at least one of a base and a basic salt, water including an acid, or a mixture of water and a water soluble organic solvent. In addition, the "water soluble" means solubility in water with respect to the solubility.

In the present specification, a "terminal" of a "terminal hydrophobic group" means a moiety other than the repeating unit constituting the polymer.

In the present specification, a "mass-average molecular weight" means the mass-average molecular weight (sodium polystyrene sulfonate-equivalent or polyethylene glycol-equivalent) measured by gel permeation chromatography (GPC).

In the present specification, a numerical value range represented using "to" means a range including the numerical values listed before and after "to" as the lower limit value and the upper limit value.

### [Water Soluble Composition Including Water Soluble Polymer]

An embodiment of the present invention is a water soluble composition including a water soluble polymer and satisfying the following Conditions (A1) and (A2) (hereinafter also referred to as a "water soluble composition (i)").
(A1) The water soluble polymer includes a nitrogen-containing functional group.
(A2) A 1.0% by mass aqueous solution of the water soluble composition (i) has a surface tension at 25°C of 34.0 to 50.0.

The surface tension in Condition (A2) is measured by the following method.

The surface tension of the 1.0% by mass aqueous solution is a value obtained by dissolving the water soluble composition in water to give a concentration of 1.0% by mass, adjusting the obtained aqueous solution (1.0% by mass aqueous solution) to 25°C, and measuring the surface tension of the aqueous solution by a plate method (Wilhelmy method) using an automatic tensiometer. Specifically, a measurement element (platinum plate) is immersed in a 1.0% by mass aqueous solution adjusted to 25°C, and the surface tension is determined from the displacement of the measurement element submerged in the 1.0% by mass aqueous solution when the force (surface tension) of the measurement element pulled by the 1.0% by mass aqueous solution is balanced with the force of a spring fixing the measurement element.

The water soluble composition (i) may be in Aspect 1 or Aspect 2.

Aspect 1: A water soluble composition that includes a water soluble polymer having interfacial performance, and satisfies Condition (A1) and Condition (A2).

Aspect 2: A water soluble composition that includes a water soluble polymer satisfying Condition (A1) and a surfactant satisfying Condition (A2).

The water soluble composition (i) of the present embodiment preferably further satisfies Condition (A3), and more preferably further satisfies Condition (A4).
(A3) The water soluble composition includes more than 0 ppm by mass and 1.3 ppm by mass or less of a component quantified by the following measurement method (hereinafter also referred to as a "component (X)").
(A4) The water soluble composition includes more than 0 ppm by mass and less than 1.0 ppm by mass of a component quantified by the following measurement method.

That is, in the water soluble composition (i), the contained amount of the component (X), quantified by the following measurement method, is preferably more than 0 ppm by mass and 1.3 ppm by mass or less, and more preferably more than 0 ppm by mass and less than 1.0 ppm by mass with respect to the total mass of the water soluble composition (i).

### <Measurement Method>

A sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition (i) is dissolved in a mixed solvent of water and methanol at a volume ratio (water/methanol) of 2/8 to give a concentration of 0.2% by mass, such that the volume ratio (solution (I)/solution (II)) is 1/10.

After injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan. That is, the area of the peak detected while the column oven is maintained at 300°C for 10 minutes after the temperature reaching 300°C is quantified by an internal standard method in which n-dodecyl mercaptan is used as a standard sample and ethyl n-caproate is used as an internal standard substance, and the value of the area is defined as a contained amount (concentration) of the component (X) in the surfactant.

Furthermore, the peak area is an area of a part surrounded by a peak, with a baseline connecting a rising point of the peak (a starting point of the peak) and an ending point of the peak (an ending point of the peak) with a straight line.

The calibration curve is created as follows.

First, n-dodecyl mercaptan is dissolved in a mixed solvent of water and methanol in a volume ratio (water/methanol) of 2/8 to give concentrations of each of 500 mg/L, 100 mg/L, 50 mg/L, and 10 mg/L to prepare solutions (III-1) to (III-4) (which are hereinafter also collectively referred to as a "solution (III)"). Each of samples (S2) to (S5) obtained by mixing each of the solutions (III-1) to (III-4) with the solution (I) such that the volume ratio (solution (I)/solution (III)) is 1/10 is subjected to gas chromatography analysis.

After injecting each of the samples (S2) to (S5) into gas chromatography, in 35-minute analysis in which a time point at which the column oven is heated to 50°C is set as 0 minutes and the temperature is raised to 300°C at 10°C/minute and maintained at 300°C for 10 minutes, each peak area obtained between 25 minutes and 35 minutes is determined, and a calibration curve showing a relationship between the concentration ratio of n-dodecyl mercaptan to ethyl n-caproate as an internal standard substance and the peak area ratio is created.

The component (X) is a defect factor component, and is considered to be a chain transfer agent remaining in the water soluble polymer, which is mainly used in the production of the water soluble polymer. When the contained amount of the component (X) is equal to or less than the upper limit value, the proportion of the defect factor component included in the water soluble composition (i) is small, and thus foreign matter is less likely to be generated in a coating film formed of the conductive composition including the water soluble composition (i) of the present embodiment.

The lower the contained amount of the component (X), the lower the proportion of the defect factor component included in the water soluble composition (i). Therefore, the smaller the contained amount of the component (X) is, the more preferable it is. Specifically, the contained amount of the component (X) is more than 0 ppm by mass and 1.3 ppm by mass or less, preferably more than 0 ppm by mass and 1.2 ppm by mass or less, more preferably more than 0 ppm by mass and 1.0 ppm by mass or less, and still more preferably more than 0 ppm by mass and 0.8 ppm by mass or less with respect to the total mass of the water soluble composition (i).

Another embodiment of the present invention is a water soluble composition including a water soluble polymer and satisfying the following (B1) to (B3) (hereinafter also referred to as a "water soluble composition (ii)").
(B1) The water soluble polymer includes a nitrogen-containing functional group.
(B2) A 1.0% by mass aqueous solution of the water soluble composition (ii) has a surface tension at 25°C of 10.0 to 50.0.
(B3) The water soluble composition (ii) includes more than 0 ppm by mass and 1.3 ppm by mass or less of the component (X).

The method for measuring the surface tension under Condition (B2) and the method for measuring the contained amount of the quantitative component under Condition (B3) are each the same as the method for measuring the surface tension under Condition (A2) and the method for measuring the contained amount of the component (X) under Condition (A3), except that the measurement target is the water soluble composition (ii).

The water soluble composition (ii) may be in Aspect 1 or Aspect 2.

Aspect 1: A water soluble composition that includes a water soluble polymer having interfacial performance, and satisfies Condition (B1) and Condition (B2).

Aspect 2: A water soluble composition that includes a water soluble polymer satisfying Condition (B1) and a surfactant satisfying Condition (B2).

The component (X) in Condition (B3) is a defect factor component, and is considered to be a chain transfer agent remaining in the water soluble polymer, which is mainly used in the production of the water soluble polymer. When the contained amount of the component (X) is equal to or less than the upper limit value, the proportion of the defect factor component included in the water soluble composition (ii) is small, and thus foreign matter is less likely to be generated in a coating film formed of the conductive composition including the water soluble composition (ii) of the present embodiment. The lower the contained amount of the component (X), the lower the proportion of the defect factor component included in the water soluble composition (ii). Therefore, the smaller the contained amount of the component (X) is, the more preferable it is, and specifically, the contained amount of the component (X) is more than 0 ppm by mass and 1.3 ppm by mass or less, preferably more than 0 ppm by mass and 1.2 ppm by mass or less, more preferably more than 0 ppm by mass and 1.0 ppm by mass or less, and still more preferably more than 0 ppm by mass and 0.8 ppm by mass or less with respect to the total mass of the water soluble composition (ii).

As the water soluble polymer, preferred is a compound which has a main chain structure of a homopolymer of a vinyl monomer having a nitrogen-containing functional group or a copolymer of a vinyl monomer having a nitrogen-containing functional group and a vinyl monomer not having a nitrogen-containing functional group (other vinyl monomer), and which has a hydrophobic group at a moiety other than the repeating unit constituting the polymer. Among these, in particular, a compound including a pyrrolidone structure, that is, a compound including a unit derived from pyrrolidone is more preferable.

Examples of the nitrogen-containing functional group include an amide group, an imide group, an amino group, a group derived from an aromatic heterocyclic structure, a group derived from a non-aromatic heterocyclic structure, and a cyano group. Among these, from the viewpoint of the solubility, the amide group is preferable. The amide group may be cyclic.

Examples of the vinyl monomer having a nitrogen-containing functional group include acrylamide and derivatives thereof, and heterocyclic monomers having a nitrogen-containing functional group, and among which those having an amide bond are preferable. Specific examples thereof include acrylamide, N,N-dimethylacrylamide, N-isopropylacrylamide, N,N-diethylacrylamide, N,N-dimethylaminopropylacrylamide, t-butylacrylamide, diacetoneacrylamide, N,N'-methylenebisacrylamide, N-vinyl-N-methylacrylamide, N-vinylpyrrolidone, and N-vinylcaprolactam. Among these, from the viewpoint of solubility, acrylamide, N-vinylpyrrolidone, N-vinylcaprolactam, or the like is preferable, and N-vinylpyrrolidone is particularly preferable.

Other vinyl monomers are not particularly limited as long as the vinyl monomers can be copolymerized with vinyl monomers having a nitrogen-containing functional group, and examples thereof include styrene, acrylic acid, vinyl acetate, and long-chain α-olefins.

These water soluble polymers may be used alone or a mixture of two or more kinds thereof may be used in any proportion.

The main chain part of the water soluble polymer is water soluble and has a nitrogen-containing functional group. The number of units (degree of polymerization) of the main chain part is preferably 2 to 1,000, more preferably 2 to 100, and particularly preferably 2 to 50, in one molecule. By setting the number of units of the main chain part having a nitrogen-containing functional group to be within this range, it is easy to control the interfacial performance.

The mass-average molecular weight of the water soluble polymer in terms of polyethylene glycol by GPC is preferably 600 or more, more preferably 1,000 or more, and still more preferably 1,500 or more, and is preferably 1,000,000 or less, more preferably 100,000 or less, still more preferably 20,000 or less, even still more preferably 10,000 or less, particularly preferably less than 2,000, and most preferably 1,800 or less. The lower limit and the upper limit of the mass-average molecular weight of the water soluble polymer can be optionally combined, and the mass-average molecular weight of the water soluble polymer is, for example, preferably 600 to 1,000,000, more preferably 600 to 100,000, still more preferably 600 to 20,000, even still more preferably 600 or more and 10,000 or less, particularly preferably 600 or more and less than 2,000, and most preferably 600 to 1,800. When the mass-average molecular weight of the water soluble polymer is equal to or more than the lower limit value, the coating properties of the conductive composition are increased. On the other hand, when the mass-average molecular weight of the water soluble polymer is equal to or less than the upper limit value, the water solubility of the conductive composition is increased. In particular, when the mass-average molecular weight of the water soluble polymer is 600 or more and 10,000 or less, the balance between the practical water solubility and the coating properties is excellent.

The surfactant is not particularly limited as long as the contained amount of the component (X) in the water soluble composition is within the range, and examples thereof include an anionic surfactant, a cationic surfactant, an amphoteric surfactant, and a non-ionic surfactant.

The molecular weight of the surfactant is preferably 2,000 or less, more preferably 1,000 or less, and still more preferably less than 600. In addition, the molecular weight of the surfactant is preferably 50 or more, and more preferably 100 or more. The lower limit and the upper limit of the molecular weight of the surfactant can be optionally combined, and the molecular weight is, for example, preferably 50 or more and 2,000 or less, more preferably 50 or more and 1,000 or less, and still more preferably 100 or more and less than 600.

The surface tension of a 1.0% by mass aqueous solution of the surfactant at 25°C is preferably 10 or more, more preferably 20 or more, still more preferably 30 or more, and particularly preferably 35 or more. In addition, the surface tension of the 1.0% by mass aqueous solution of the surfactant at 25°C is preferably 50 or less, more preferably 45 or less, and still more preferably 40 or less. The surface tension of the surfactant can be optionally combined, and is, for example, preferably 10 to 50, more preferably 20 to 50, still more preferably 30 to 45, and particularly preferably 35 to 40.

These surfactants may be used alone or a mixture of two or more kinds thereof may be used in any proportion.

Examples of the anionic surfactants include sodium dialkylsulfosuccinate, sodium octanoate, sodium decanoate, sodium laurate, sodium myristate, sodium palmitate, sodium stearate, perfluorononanoic acid, N-sodium lauroyl sarcosine, sodium cocoyl glutamate, alpha sulfo fatty acid methyl ester salts, sodium lauryl sulfate, sodium myristyl sulfate, sodium laureth sulfate, sodium polyoxyethylene alkyl phenol sulfonate, ammonium lauryl sulfate, lauryl phosphate, sodium lauryl phosphate, and potassium lauryl phosphate.

Examples of the cationic surfactants include tetramethylammonium chloride, tetramethylammonium hydroxide, tetrabutylammonium chloride, dodecyldimethylbenzylammonium chloride, alkyltrimethylammonium chloride, octyltrimethylammonium chloride, decyltrimethylammonium chloride, dodecyltrimethylammonium chloride, tetradecyltrimethylammonium chloride, cetyltrimethylammonium chloride, stearyltrimethylammonium chloride, alkyltrimethylammonium bromide, hexadecyltrimethylammonium bromide, benzyltrimethylammonium chloride, benzyltriethylammonium chloride, benzalkonium chloride, benzalkonium bromide, benzethonium chloride, dialkyl dimethylammonium chloride, didecyl dimethylammonium chloride, distearyl dimethylammonium chloride, monomethylamine hydrochloride, dimethylamine hydrochloride, trimethylamine hydrochloride, butylpyridinium chloride, dodecylpyridinium chloride, and cetylpyridinium chloride.

Examples of the amphoteric surfactants include lauryl dimethylamino acetate betaine, stearyl dimethylamino acetate betaine, dodecylamino methyl dimethyl sulfopropyl betaine, octadecylamino methyl dimethyl sulfopropyl betaine, cocamidopropyl betaine, cocamidopropyl hydroxysultaine, 2-alkyl-N-carboxymethyl-N-hydroxyethyl imidazolinium betaine, sodium lauroyl glutamate, potassium lauroyl glutamate, lauroyl methyl-β-alanine, lauryl dimethylamine N-oxide, and oleyl dimethylamine N-oxide.

Examples of the non-ionic surfactants include glycerol laurate, glycerol monostearate, sorbitan fatty acid esters, sucrose fatty acid esters, polyoxyethylene alkyl ethers, pentaethylene glycol monododecyl ether, octaethylene glycol monododecyl ether, polyoxyethylene alkyl phenyl ether, octylphenol ethoxylate, nonylphenol ethoxylate, polyoxyethylene polyoxypropylene glycol, polyoxyethylene sorbitan fatty acid esters, polyoxyethylene hexitan fatty acid esters, sorbitan fatty acid esters polyethylene glycol, lauric acid diethanolamide, oleic acid diethanolamide, stearic acid diethanolamide, octyl glucoside, decyl glucoside, lauryl glucoside, cethanol, stearyl alcohol, and oleyl alcohol.

As the non-ionic surfactant, a water soluble polymer having interfacial performance may be used in addition to the above-described surfactants.

As in Aspect 1, from the viewpoint of enhancing the coating properties, it is preferable that the water soluble polymer having interfacial performance has a nitrogen-containing functional group and further has a terminal hydrophobic group. Unlike the surfactants of the related art, water soluble polymers having nitrogen-containing functional groups and terminal hydrophobic groups can have interfacial performance due to the main chain part (hydrophilic part) having nitrogen-containing functional groups and the terminal hydrophobic group part and are particularly highly effective in improving the coating properties.

Examples of the terminal hydrophobic group include alkyl groups, aralkyl groups, aryl groups, alkoxy groups, aralkyloxy groups, aryloxy groups, alkylthio groups, aralkylthio groups, arylthio groups, primary or secondary alkylamino groups, aralkylamino groups, and arylamino groups. Among these, the alkylthio groups, the aralkylthio groups, and the arylthio groups are preferable.

The number of carbon atoms of the terminal hydrophobic group is preferably 7 to 100, more preferably 7 to 50, and particularly preferably 10 to 30.

The number of the terminal hydrophobic groups in the water soluble polymer is not particularly limited. In addition, when two or more terminal hydrophobic groups are contained in the same molecule, the terminal hydrophobic groups may be of the same kind or may be of different kinds.

A method for introducing a terminal hydrophobic group into a water soluble polymer is not particularly limited, but usually, introduction by selecting a chain transfer agent during vinyl polymerization is convenient and preferable. In this case, the chain transfer agent is not particularly limited as long as a group including a hydrophobic group such as an alkyl group, an aralkyl group, an aryl group, an alkylthio group, an aralkylthio group, or an arylthio group is introduced at a terminal of the obtained polymer. For example, when obtaining a water soluble polymer having an alkylthio group, an aralkylthio group, or an arylthio group as a terminal hydrophobic group, it is preferable to perform the vinyl polymerization using a chain transfer agent having a hydrophobic group corresponding to the terminal hydrophobic group, specifically a thiol, a disulfide, a thioether, or the like. Examples of such a chain transfer agent include n-dodecyl mercaptan, t-dodecyl mercaptan, n-octyl mercaptan, 2-ethylhexyl mercaptan, and n-octadecyl mercaptan.

These may be used alone or a combination of two or more kinds thereof may be used.

As a polymerization initiator used during the vinyl polymerization, an azobis-methylbutyronitrile or a polymerization initiator having a terminal hydrophobic group can be used.

Examples of the polymerization initiator having a terminal hydrophobic group include those having a linear or branched alkyl group having 6 to 20 carbon atoms and not having a cyano group and a hydroxy group, and specific examples thereof include dilauroyl peroxide, 1,1,3,3-tetramethylbutylhydroperoxide, di-t-hexyl peroxide, di(2-ethylhexyl)peroxydicarbonate, 1,1,3,3-tetramethylbutylperoxyneodecanoate, and di(3,5,5-trimethylhexanoyl)peroxide. These may be used alone or a combination of two or more kinds thereof may be used.

A molecular weight ratio of the main chain part in the water soluble polymer to the terminal hydrophobic group part (for example, an alkyl group, an aralkyl group, an aryl group, an alkylthio group, an aralkylthio group, and an arylthio group) (the mass-average molecular weight of the main chain part/the mass-average molecular weight of the terminal hydrophobic group part) is preferably 0.3 to 170.

The surface tension at 25°C of the 1.0% by mass aqueous solution of the water soluble composition including the water soluble polymer having interfacial performance in Aspect 1 and the water soluble composition including the water soluble polymer and the surfactant in Aspect 2 is preferably 10 or more, more preferably 20 or more, still more preferably 30 or more, and particularly preferably 35 or more. In addition, the surface tension of the 1.0% by mass aqueous solution of the water soluble polymer having interfacial performance at 25°C is preferably 50 or less, more preferably 45 or less, and still more preferably 40 or less. The lower limit and the upper limit of the surface tension can be optionally combined, and the surface tension is, for example, preferably 10 to 50, more preferably 20 to 50, still more preferably 30 to 45, and particularly preferably 35 to 40.

When the water soluble polymer and the surfactant are used in combination as in Aspect 2, the water soluble polymer may be a water soluble polymer having interfacial performance or may be a water soluble polymer in which the 1.0% by mass aqueous solution has a surface tension at 25°C of more than 50.

Examples of the water soluble polymer in which the 1.0% by mass aqueous solution has a surface tension at 25°C of more than 50 include a water soluble polymer having a nitrogen-containing functional group and not having a terminal hydrophobic group.

As the surfactant, from those described above, the non-ionic surfactant is preferable from the viewpoint of a less effect on the resist layer, and among the non-ionic surfactants, in particular, the water soluble polymer having a nitrogen-containing functional group is preferable, and the water soluble polymer having a nitrogen-containing functional group and a terminal hydrophobic group is more preferable. Among the water soluble polymers, in particular, a compound represented by General Formula (7) (hereinafter also referred to as a "compound (7)") or a compound represented by General Formula (8) (hereinafter also referred to as a "compound (8)") is preferable. These compounds may be used alone or a mixture of two or more kinds thereof may be used in any proportion.

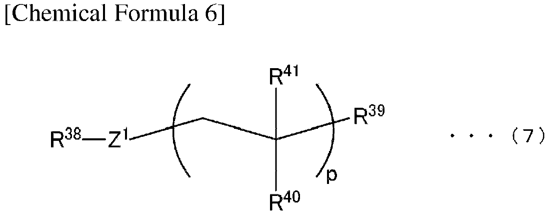

In General Formula (7), R³⁸ represents a hydrogen atom, a linear or branched alkyl group, an aralkyl group, or an aryl group, R³⁹ represents a hydrogen atom, an alkylthio group, an aralkylthio group, an arylthio group, or a hydrocarbon group, R⁴⁰ represents a hydrophilic group, R⁴¹ represents a hydrogen atom or a methyl group, Z¹ represents a single bond, -S-, -S(=O)-, -C(=O)-O-, or -O-, and p is a number of 1 to 50. It should be noted that R³⁸ and R³⁹ are not hydrogen atoms at the same time.

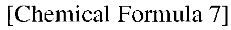

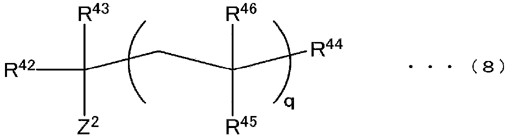

In General Formula (8), R⁴² and R⁴³ each independently represent a methyl group or an ethyl group, R⁴⁴ represents a hydrogen atom, an alkylthio group, an aralkylthio group, an arylthio group, or a hydrocarbon group, R⁴⁵ represents a hydrophilic group, R⁴⁶ represents a hydrogen atom or a methyl group, Z² represents a cyano group or a hydroxy group, and q is a number of 1 to 50.

The number of carbon atoms in the alkyl group in R³⁸ is preferably 6 to 20.

The number of carbon atoms in the aralkyl group in R³⁸ is preferably 7 to 20.

The number of carbon atoms in the aryl group in R³⁸ is preferably 6 to 20.

Examples of the alkylthio group in R³⁹ and R⁴⁴ include a group in which a sulfur atom is bonded to a linear or branched alkyl group having 1 to 20 carbon atoms.

Examples of the aralkylthio group in R³⁹ and R⁴⁴ include a group in which a sulfur atom is bonded to a linear or branched aralkyl group having 7 to 20 carbon atoms.

Examples of the aryl group in R³⁹ and R⁴⁴ include a group in which a sulfur atom is bonded to a linear or branched aryl group having 6 to 20 carbon atoms.

Examples of the hydrocarbon group in R³⁹ and R⁴⁴ include a linear or branched alkyl group having 1 to 20 carbon atoms, a linear or branched alkenyl group having 1 to 20 carbon atoms, and a linear or branched alkynyl group having 1 to 20 carbon atoms.

The hydrophilic group in R⁴⁰ and R⁴⁵ is derived from a water soluble vinyl monomer which is a raw material monomer of the water soluble polymer. Here, the water soluble vinyl monomer indicates a vinyl monomer that can be mixed with water in any proportion. Examples of such a water soluble vinyl monomer include N-vinylpyrrolidone, 2-hydroxyethyl (meth)acrylate, (meth)acrylamide, N,N-dimethylacrylamide, acryloyl morpholine, and N-vinylformamide.

Examples of the compound (7) include a compound represented by General Formula (71) (hereinafter also referred to as a "compound (71)").

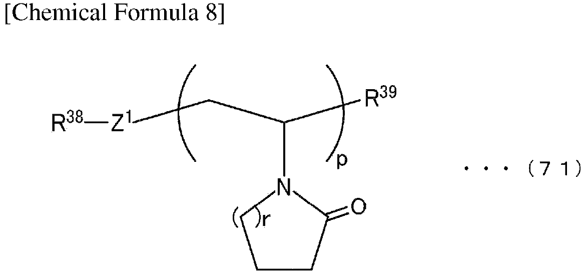

In General Formula (71), R³⁸ represents a hydrogen atom, a linear or branched alkyl group, an aralkyl group, or an aryl group, R³⁹ represents a hydrogen atom, an alkylthio group, an aralkylthio group, an arylthio group, or a hydrocarbon group, Z¹ represents a single bond, -S-, -S(=O)-, -C(=O)-O-, or -O-, p is a number of 1 to 50, and r is a number of 1 to 5. It should be noted that R³⁸ and R³⁹ are not hydrogen atoms at the same time.

Examples of the compound (8) include a compound represented by General Formula (81) (hereinafter also referred to as a "compound (81)").

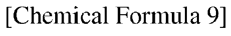

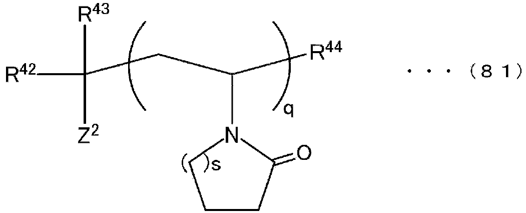

In General Formula (81), R⁴² and R⁴³ each independently represent a methyl group or an ethyl group, R⁴⁴ represents a hydrogen atom, an alkylthio group, an aralkylthio group, an arylthio group, or a hydrocarbon group, Z² represents a cyano group or a hydroxy group, q is a number of 1 to 50, and s is a number of 1 to 5.

### <Method for Producing Water Soluble Composition>

One embodiment of the present invention is a method for producing a water soluble composition. The production method which will be described later is preferably used for producing a water soluble composition including a water soluble polymer having a nitrogen-containing functional group.

The water soluble composition in which the contained amount of the component (X) is within the range is obtained by purifying the water soluble polymer.

Examples of a method for purifying the water soluble polymer include a reprecipitation method, a membrane filtration method with a filter, an ion exchange method, washing with a solvent, recrystallization, and liquid extraction. Among these, it is particularly preferable to purify the surfactant by a reprecipitation method. The reprecipitation method is a method in which a purification target is dissolved in a good solvent having high solubility of the purification target to prepare a good solvent solution, and the good solvent solution is mixed with a poor solvent having low solubility of the purification target to purify the purification target.

In addition, the water soluble polymer may be purified by combination of two or more of these methods.

Hereinafter, an example of a method for producing a water soluble polymer by a reprecipitation method will be described.

The method for producing a water soluble composition of the present embodiment includes a reprecipitation step of mixing a good solvent solution including a water soluble polymer with an ether.

Specifically, a good solvent solution is prepared by dissolving a water soluble polymer in a good solvent, and the good solvent solution and a poor solvent are mixed to purify the water soluble polymer.

The good solvent used in the good solvent solution is not particularly limited as long as it is a solvent in which the water soluble polymer has high solubility. Examples of the good solvent for the water soluble polymer include alcohols such as methanol, ethanol, isopropyl alcohol, propyl alcohol, and butanol; ketones such as acetone, methyl ethyl ketone, methyl isobutyl ketone, and propylene glycol monomethyl ether acetate; esters such as ethyl acetate and butyl acetate; nitriles such as acetonitrile; halogenated alkyls such as chloroform and dichloromethane; aromatics such as toluene and benzene; nitrogen-containing compounds such as dimethylformamide and N-methyl-2-pyrrolidone; and sulfur-containing compounds such as dimethyl sulfoxide. Among these, the alcohols are preferable from the viewpoint of ease of removal due to high polarity and a low boiling point. These good solvents may be used alone or a mixture of two or more kinds thereof may be used in any proportion.

Furthermore, the expression "high solubility of the water soluble polymer" means that 0.1 g or more of the water soluble polymer is uniformly dissolved in 10 g of a solvent (liquid temperature: 25°C).

The poor solvent is not particularly limited as long as it is a solvent in which the solubility of the water soluble polymer is low. From the viewpoint of improving the purification efficiency, as the poor solvent for the water soluble polymer, the ether is preferable, and the ether and the alkane are more preferably used in combination. That is, in the reprecipitation step, it is preferable to mix the good solvent solution with the ether, and it is more preferable to mix the good solvent solution with the ether and the alkane.

Furthermore, the "solubility of the water soluble polymer is low" means that the amount of the water soluble polymer dissolved in 10 g of a solvent (liquid temperature: 25°C) is less than 0.1 g.

The ether is a solvent in which two carbon atoms are bonded to one oxygen atom.

Examples of the ether include diethyl ether, diisopropyl ether, t-butyl methyl ether, methoxycyclopentene, and dibutyl ether. Among these, diethyl ether, diisopropyl ether, or t-butyl methyl ether is preferable from the viewpoint of low polarity and ease of removal due to a low boiling point.

These ethers may be used alone or a mixture of two or more kinds thereof may be used in any proportion.

The alkane is a solvent consisting of a linear or branched saturated hydrocarbon.

Examples of the alkane include pentane, hexane, heptane, cyclopentane, methylcyclohexane, octane, nonane, decane, undecane, and dodecane. Among these, from the viewpoint of enhancing the purification efficiency, an alkane having 1 to 20 carbon atoms is preferable, and among these, from the viewpoint of particularly ease of removal due to low polarity and a low boiling point, an alkane having 5 to 10 carbon atoms is more preferable, and hexane is still more preferable. These alkanes may be used alone or a mixture of two or more kinds thereof may be used in any proportion.

The contained amount of the water soluble polymer with respect to the total mass of the good solvent solution is preferably 40% to 80% by mass, more preferably 50% to 75% by mass, and still more preferably 55% to 70% by mass.

The mixed amount of the poor solvent is preferably 100 parts by mass or more, more preferably 500 parts by mass or more, and still more preferably 1,000 parts by mass or more, and is preferably 10,000 parts by mass or less, more preferably 8,000 parts by mass or less, and still more preferably 7,000 parts by mass or less with respect to 100 parts by mass of the good solvent solution. The lower limit and the upper limit of the mixed amount of the poor solvent can be optionally combined, and the mixed amount of the poor solvent is, for example, more preferably 100 to 10,000 parts by mass, still more preferably 500 to 8,000 parts by mass, and most preferably 1,000 to 7,000 parts by mass. When the mixed amount of the poor solvent is equal to or more than the lower limit value, the yield of the water soluble polymer is increased. When the mixed amount of the poor solvent is equal to or less than the upper limit value, the purification efficiency is further increased.

When an ether and an alkane are used in combination as the poor solvent, the mass ratio of the alkane to the ether (alkane/ether) is preferably 0.5 or more, more preferably 0.7 or more, and still more preferably 0.8 or more, and is preferably 2.0 or less, more preferably 1.6 or less, and still more preferably 1.4 or less. The lower limit and the upper limit of the alkane/ether can be optionally combined, and the ratio is, for example, preferably 0.5 to 2.0, more preferably 0.7 to 1.6, and still more preferably 0.8 to 1.4. When the alkane/ether ratio is equal to or more than the lower limit value, the yield is easily improved. When the alkane/ether ratio is equal to or less than the upper limit value, the defect factor component is easily removed.

The good solvent solution is mixed with the poor solvent to precipitate the water soluble polymer in the good solvent solution. By filtering the precipitate, the surfactant is obtained in a state of wet powder. By drying the wet powder, a dry powder of the water soluble polymer is obtained.

In this way, a water soluble polymer from which impurities such as the component (X) are sufficiently removed is obtained.

The reprecipitation step may be performed once or twice or more. In particular, when the component (X) is not sufficiently removed in one reprecipitation step, the reprecipitation step is repeated until the contained amount of the component (X) in the water soluble polymer is within the range.

When the reprecipitation step is performed twice or more, an operation of preparing a good solvent solution by dissolving the wet powder obtained in the immediately preceding reprecipitation step again in a good solvent, mixing the good solvent solution with a poor solvent, and separating the obtained precipitate by filtration is repeated.

The method for producing a water soluble composition of the present embodiment may further include a synthesis step for a water soluble polymer before the reprecipitation step, and the reaction solution obtained in the synthesis step may be used as a good solvent solution in the reprecipitation step. That is, the water soluble polymer may be synthesized by performing polymerization using the solvent exemplified as the good solvent described above, and in the reprecipitation step, the poor solvent may be mixed with the reaction solution after the synthesis of the water soluble polymer to perform reprecipitation purification.

### <Action Effect>

Since the water soluble composition including the water soluble polymer of the present embodiment described above satisfies Condition (A1) and (A2) or satisfies the Conditions (B1) to (B3), the defect factor component is sufficiently reduced.

Therefore, the use of the water soluble composition of the present embodiment makes it possible to obtain a conductive composition that has favorable coating properties and is less likely to generate foreign matter during formation of a coating film.

### [Conductive Composition]

An embodiment of the present invention is a conductive composition.

The conductive composition of the present embodiment is a composition including a conductive polymer (A) shown below and the above-described water soluble composition of the present invention (hereinafter also referred to as a "water soluble composition (C)").

The conductive composition may further include at least one selected from a basic compound (B) and a solvent (D) shown below, in addition to the conductive polymer (A) and the water soluble composition (C). In addition, the conductive composition may further include a component (optional component) other than the conductive polymer (A), the basic compound (B), the water soluble composition (C), and the solvent (D). **In** the present embodiment, the surface tension of the 1.0% by mass aqueous solution at 25°C of Condition (A2) and Condition (B2) satisfied by the water soluble composition is applied as a condition satisfied by the conductive composition.

In the present embodiment, the contained amount of the specific component of Conditions (A3), (A4), (B3), and (B4) satisfied by the water soluble composition is applied as a condition satisfied by the conductive composition.

### <Conductive Polymer (A)>

As the conductive polymer (A), known polymers can be used, and examples thereof include polythiophene, polythiophene vinylene, poly(3-alkylthiophene), poly(3,4-ethylenedioxy)thiophene (PEDOT), poly(3,4-ethylenedioxythiophene)-polystyrenesulfonic acid (PEDOT-PSS), polyaniline, polypyrrole, polytellurophene, polyparaphenylene, polyparaphenylenevinylene, polyacene, and polyacetylene.

The conductive polymer (A) is preferably water soluble or water dispersible. When the conductive polymer (A) is water soluble or water dispersible, the coating properties of the conductive composition is improved and a conductive film having a uniform thickness is easily obtained.

The conductive polymer (A) preferably has an acidic group or a salt of the acidic group. When the conductive polymer (A) has an acidic group or a salt of the acidic group, the water solubility of the conductive polymer (A) is increased.

The conductive polymer (A) may have two kinds of acidic groups in one molecule. Some or all of the acidic groups may form salts.

As the conductive polymer (A) having an acidic group or a salt of the acidic group, for example, the conductive polymers shown in Japanese Unexamined Patent Application, First Publication No. S61-197633, Japanese Unexamined Patent Application, First Publication No. S63-39916, Japanese Unexamined Patent Application, First Publication No. H1-301714, Japanese Unexamined Patent Application, First Publication No. H5-504153, Japanese Unexamined Patent Application, First Publication No. H5-503953, Japanese Unexamined Patent Application, First Publication No. H4-32848, Japanese Unexamined Patent Application, First Publication No. H4-328181, Japanese Unexamined Patent Application, First Publication No. H6-145386, Japanese Unexamined Patent Application, First Publication No. H6-56987, Japanese Unexamined Patent Application, First Publication No. H5-226238, Japanese Unexamined Patent Application, First Publication No. H5-178989, Japanese Unexamined Patent Application, First Publication No. H6-293828, Japanese Unexamined Patent Application, First Publication No. H7-118524, Japanese Unexamined Patent Application, First Publication No. H6-32845, Japanese Unexamined Patent Application, First Publication No. H6-87949, Japanese Unexamined Patent Application, First Publication No. H6-256516, Japanese Unexamined Patent Application, First Publication No. H7-41756, Japanese Unexamined Patent Application, First Publication No. H7-48436, Japanese Unexamined

Patent Application, First Publication No. H4-268331, Japanese Unexamined Patent Application, First Publication No. 2014-65898, or the like are preferable from the viewpoint of solubility.

Specific examples of the conductive polymer (A) include π-conjugated conductive polymers including at least one repeating unit selected from the group consisting of phenylenevinylene, vinylene, thienylene, pyrrolylene, phenylene, iminophenylene, isothianaphtene, furylene, and carbazolylen in which the α-position or β-position is substituted with at least one group selected from the group consisting of a sulfonic acid group and a carboxy group.

In addition, when the π-conjugated conductive polymer includes at least one repeating unit selected from the group consisting of iminophenylene and carbazolylene, examples of the conductive polymer include a conductive polymer having an acidic group or a salt of the acidic group on a nitrogen atom of the repeating unit, and a conductive polymer having an alkyl group substituted with an acidic group or a salt of the acidic group or an alkyl group including an ether bond on the nitrogen atom.

Among these, a conductive polymer having, as a monomer unit, at least one selected from the group consisting of thienylene, pyrrolylene, iminophenylene, phenylenevinylene, carbazolylene, and isothianaphthene, the β-position of which is substituted with an acidic group or a salt of the acidic group, is preferably used from the viewpoint of conductivity and solubility.

Among the above, from the viewpoint of excellent conductivity, polyaniline having an acidic group or a salt of the acidic group, PEDOT, PEDOT-PSS, polypyrrole, or polythiophene is preferable as the conductive polymer (A). Among these, in particular, the polyaniline having an acidic group or a salt of the acidic group is particularly suitable as the conductive polymer since it is a compound having excellent solubility in water.

Examples of the polyaniline having an acidic group or a salt of the acidic group include a polymer having an acidic group or a salt of the acidic group on a skeleton of a π-conjugated polymer such as unsubstituted or substituted polyaniline or polydiaminoanthraquinone or on a nitrogen atom in the π-conjugated polymer.

Among these, the conductive polymer having at least one unit selected from units represented by General Formulae (1) to (4) is preferable, and a conductive polymer containing 20% to 100% by mole of the unit in all the units (100% by mole) constituting the conductive polymer is more preferable from the viewpoint that high conductivity and solubility can be exhibited.

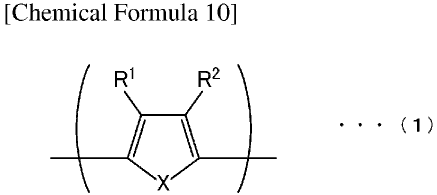

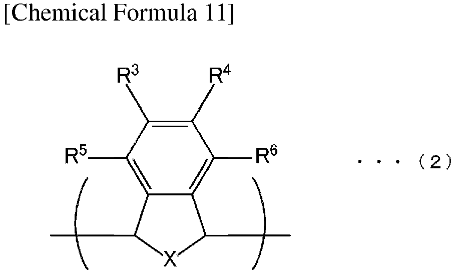

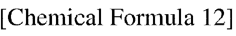

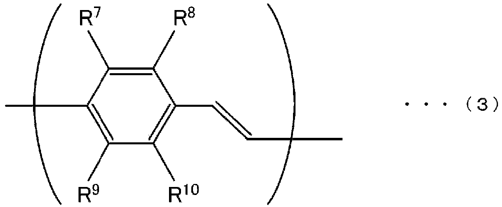

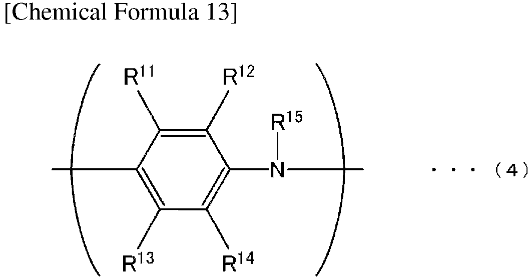

In General Formulae (1) to (4), X represents a sulfur atom or a nitrogen atom, and R¹ to R¹⁵ each independently represent a hydrogen atom, a linear or branched alkyl group having 1 to 24 carbon atoms, a linear or branched alkoxy group having 1 to 24 carbon atoms, an acidic group or a salt of the acidic group, a hydroxy group, a nitro group, a halogen atom (-F, -Cl, -Br, or -I), -N(R¹⁶)₂, -NHCOR¹⁶, -SR¹⁶, -OCOR¹⁶, - COOR¹⁶, -COR¹⁶, -CHO, or -CN. R¹⁶ represents an alkyl group having 1 to 24 carbon atoms, an aryl group having 6 to 24 carbon atoms, or an aralkyl group having 7 to 24 carbon atoms.

From the viewpoint of controlling water solubility, it is preferable that at least one of R¹ and R² in General Formula (1), at least one of R³ to R⁶ in General Formula (2), at least one of R⁷ to R¹⁰ in General Formula (3), and at least one of R¹¹ to R¹⁵ in General Formula (4) are each an acidic group or a salt of the acidic group among the compounds represented by the general formulae.

Here, the "acidic group" means a sulfonic acid group (sulfo group) or a carboxylic acid group (carboxy group).

The sulfonic acid group may be included in an acidic state (-SO₃H) or may be contained in an ionic state (-SO₃-). Furthermore, the sulfonic acid group also includes a substituent having a sulfonic acid group (-R¹⁷SO₃H).

On the other hand, the carboxylic acid group may be included in an acidic state (-COOH) or may be included in an ionic state (-COO-). Furthermore, the carboxylic acid group also includes a substituent having a carboxylic acid group (-R¹⁷COOH). R¹⁷ represents a linear or branched alkylene group having 1 to 24 carbon atoms, a linear or branched arylene group having 6 to 24 carbon atoms, or a linear or branched aralkylene group having 7 to 24 carbon atoms.

As the acidic group, the sulfonic acid group is preferable.

Examples of the salt of the acidic group include alkali metal salts, alkaline earth metal salts, ammonium salts, or substituted ammonium salts of a sulfonic acid group or carboxylic acid group.

Examples of the alkali metal salts include lithium sulfate, lithium carbonate, lithium hydroxide, sodium sulfate, sodium carbonate, sodium hydroxide, potassium sulfate, potassium carbonate, potassium hydroxide, and derivatives having skeletons thereof.

Examples of the alkaline earth metal salts include a magnesium salt and a calcium salt.

Examples of the substituted ammonium salts include aliphatic ammonium salts, saturated alicyclic ammonium salts, and unsaturated alicyclic ammonium salts.

Examples of the aliphatic ammonium salts include methyl ammonium, dimethyl ammonium, trimethyl ammonium, ethyl ammonium, diethyl ammonium, triethyl ammonium, methyl ethyl ammonium, diethyl methyl ammonium, dimethyl ethyl ammonium, propylammonium, dipropylammonium, isopropylammonium, diisopropylammonium, butylammonium, dibutylammonium, methylpropylammonium, ethylpropylammonium, methylisopropylammonium, ethylisopropylammonium, methylbutylammonium, ethylbutylammonium, tetramethylammonium, tetramethylolammonium, tetraethylammonium, tetra-n-butylammonium, tetra-sec-butylammonium, and tetra t-butylammonium.

Examples of the saturated alicyclic ammonium salts include piperidinium, pyrrolidinium, morpholinium, piperazinium, and derivatives having skeletons thereof.

Examples of the unsaturated alicyclic ammonium salts include pyridinium, α-picolinium, β-picolinium, γ-picolinium, quinolinium, isoquinolinium, pyrrolinium, and derivatives having skeletons thereof.

From the viewpoint that high conductivity can be exhibited, it is preferable that the conductive polymer (A) has the unit represented by General Formula (4), and above all, particularly from the viewpoint of being also excellent in solubility, it is more preferable that the conductive polymer (A) has the unit represented by General Formula (5).

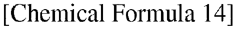

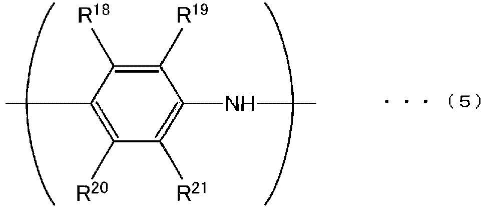

In General Formula (5), R¹⁸ to R²¹ each independently represent a hydrogen atom, a linear or branched alkyl group having 1 to 24 carbon atoms, a linear or branched alkoxy group having 1 to 24 carbon atoms, an acidic group or a salt of the acidic group, a hydroxy group, a nitro group, or a halogen atom (-F, -Cl, -Br, or -I). In addition, at least one of R¹⁸ to R²¹ is the acidic group or a salt of the acidic group.

As the unit represented by General Formula (5), it is preferable that any one of R¹⁸ to R²¹ is a linear or branched alkoxy group having 1 to 4 carbon atoms, another one is a sulfonic acid group or a salt thereof, and the remaining is a hydrogen atom from the viewpoint of ease of production.

From the viewpoints of excellent solubility in water and an organic solvent regardless of pH, the conductive polymer (A) preferably includes 10% to 100% by mole of the unit represented by General Formula (5), more preferably includes 50% to 100% by mole, and particularly preferably includes 100% by mole in all the units (100% by mole) constituting the conductive polymer (A).

In addition, the conductive polymer (A) preferably includes 10 or more of the units represented by General Formula (5) in one molecule from the viewpoint of excellent conductivity.

In addition, in the conductive polymer (A), from the viewpoint of further improving the solubility, the number of aromatic rings to which an acidic group or a salt of the acidic group is bonded with respect to the total number of aromatic rings in the polymer is preferably 50% or more, more preferably 70% or more, still more preferably 80% or more, particularly preferably 90% or more, and most preferably 100%.

The number of aromatic rings to which the acidic group or a salt of the acidic group is bonded with respect to the total number of aromatic rings in the polymer refers to a value calculated from the charging ratio of the monomer during the production of the conductive polymer (A).

In addition, in the conductive polymer (A), from the viewpoint of imparting a reactivity to a monomer, a substituent other than the acidic group or a salt of the acidic group on the aromatic ring of the monomer unit is preferably an electron donating group, and specifically, the substituent is preferably an alkyl group having 1 to 24 carbon atoms, an alkoxy group having 1 to 24 carbon atoms, a halogen group (-F, -Cl, -Br, or -I), or the like. Among these, from the viewpoint of an electron donating property, the substituent is the alkoxy group having 1 to 24 carbon atoms is most preferable.

Furthermore, the conductive polymer (A) may include, as a constitutional unit other than the unit represented by General Formula (5), one or more units selected from the group consisting of aniline, thiophene, pyrrole, phenylene, vinylene, a divalent unsaturated group, and a divalent saturated group, which is substituted or unsubstituted, as long as the solubility, the conductivity, and the properties are not affected.

From the viewpoint that high conductivity and solubility can be exhibited, the conductive polymer (A) is preferably a compound having a structure represented by General Formula (6), and among the compounds having the structure represented by General Formula (6), from the viewpoint of particularly excellent solubility, poly(2-sulfo-5-methoxy-1,4-iminophenylene) and poly(2-methoxyaniline-5-sulfonic acid) are particularly preferable.

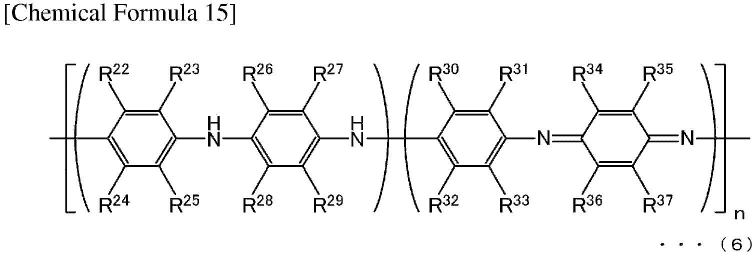

In General Formula (6), R²² to R³⁷ each independently represent a hydrogen atom, a linear or branched alkyl group having 1 to 4 carbon atoms, a linear or branched alkoxy group having 1 to 4 carbon atoms, an acidic group or a salt of the acidic group, a hydroxy group, a nitro group, or a halogen atom (-F, -Cl, -Br, or -I). In addition, at least one of R²² to R³⁷ is the acidic group or a salt of the acidic group. In addition, n represents a degree of polymerization. In the present invention, n is preferably an integer of 5 to 2,500.

As the unit represented by General Formula (6), from the viewpoint of ease of the production, it is preferable that any one of R²² to R²⁵ is a linear or branched alkoxy group having 1 to 4 carbon atoms, another is a sulfonic acid group or a salt thereof, and the remaining is a hydrogen atom, any one of R²⁶ to R²⁹ is a linear or branched alkoxy group having 1 to 4 carbon atoms, another is a sulfonic acid group or a salt thereof, and the remaining is a hydrogen atom, any one of R³⁰ to R³³ is a linear or branched alkoxy group having 1 to 4 carbon atoms, another is a sulfonic acid group or a salt thereof, and the remaining is a hydrogen atom, and any one of R³⁴ to R³⁷ is a linear or branched alkoxy group having 1 to 4 carbon atoms, another is a sulfonic acid group or a salt thereof, and the remaining is a hydrogen atom.

From the viewpoint of improving the conductivity, it is desirable that at least a part of the acidic groups contained in the conductive polymer (A) is of a free acid type.

From the viewpoint of conductivity, solubility, and film forming properties, the mass-average molecular weight of the conductive polymer (A) in terms of sodium polystyrene sulfonate by GPC is preferably 1,000 or more, more preferably 1,500 or more, and still more preferably 2,000 or more, and is preferably 1,000,000 or less, more preferably 800,000 or less, still more preferably 500,000 or less, and particularly preferably 100,000 or less. The lower limit and the upper limit of the mass-average molecular weight of the conductive polymer (A) can be optionally combined, and the mass-average molecular weight of the conductive polymer (A) is, for example, preferably 1,000 to 1,000,000, more preferably 1,500 to 800,000, still more preferably 2,000 to 500,000, and particularly preferably 2,000 to 100,000. When the mass-average molecular weight of the conductive polymer (A) is less than 1,000, the solubility is excellent, but the conductivity and the film forming properties may be lacking. On the other hand, when the mass-average molecular weight exceeds 1,000,000, the conductivity is excellent, but the solubility may be insufficient.

Here, the "film forming properties" refer to a property of forming a uniform film without cissing or the like, and can be evaluated by a method such as spin coating on glass.

### (Method for Producing Conductive Polymer (A))

The conductive polymer (A) is obtained by, for example, polymerizing raw material monomers of the conductive polymer (A) in the presence of a polymerization solvent and an oxidizing agent.

An example of a method for producing the conductive polymer (A) will be described below.

The method for producing the conductive polymer (A) of the present embodiment includes a step of polymerizing raw material monomers of the conductive polymer (A) of the present embodiment in the presence of a polymerization solvent and an oxidizing agent (polymerization step). In addition, the method for producing the conductive polymer (A) of the present embodiment may include a step of purifying a reaction product obtained in the polymerization step (purification step).

### <<Polymerization Step>>

The polymerization step is a step of polymerizing raw material monomers of the conductive polymer (A) in the presence of a polymerization solvent and an oxidizing agent.

Specific examples of the raw material monomer include a polymerizable monomer from which the above-described monomer unit is derived, and specifically include at least one selected from the group consisting of an acidic group-substituted aniline, an alkali metal salt thereof, an alkaline earth metal salt thereof, an ammonium salt thereof, and a substituted ammonium salt thereof. Examples of the acidic group-substituted aniline include a sulfonic acid group-substituted aniline having a sulfonic acid group as an acidic group.

Representative examples of the sulfonic acid group-substituted aniline include aminobenzenesulfonic acids, and specifically, o-, m-, or p-aminobenzenesulfonic acid, aniline-2,6-disulfonic acid, aniline-2,5-disulfonic acid, aniline-3,5-disulfonic acid, aniline-2,4-disulfonic acid, aniline-3,4-disulfonic acid, or the like is preferably used.

Examples of the sulfonic acid group-substituted anilines other than the aminobenzenesulfonic acids include alkyl group-substituted aminobenzenesulfonic acids such as methylaminobenzenesulfonic acid, ethylaminobenzenesulfonic acid, n-propylaminobenzenesulfonic acid, iso-propylaminobenzenesulfonic acid, n-butylaminobenzenesulfonic acid, sec-butylaminobenzenesulfonic acid, and t-butylaminobenzenesulfonic acid; alkoxy group-substituted aminobenzenesulfonic acids such as methoxyaminobenzenesulfonic acid, ethoxyaminobenzenesulfonic acid, and propoxyaminobenzenesulfonic acid; hydroxy group-substituted aminobenzenesulfonic acids; nitro group-substituted aminobenzenesulfonic acids; and halogen-substituted aminobenzenesulfonic acids such as fluoroaminobenzenesulfonic acid, chloraminobenzenesulfonic acid, and bromaminobenzenesulfonic acid.

Among these, from the viewpoint of obtaining a conductive polymer (A) having particularly excellent conductivity and solubility, the alkyl group-substituted aminobenzenesulfonic acids, the alkoxy group-substituted aminobenzenesulfonic acids, the hydroxy group-substituted aminobenzenesulfonic acids, or the halogen-substituted aminobenzenesulfonic acids are preferable, and from the viewpoint of ease of production, the alkoxy group-substituted aminobenzenesulfonic acids, the alkali metal salts thereof, ammonium salts thereof, or the substituted ammonium salts thereof are particularly preferable.

Any one of these sulfonic acid group-substituted anilines may be used alone or a mixture of two or more kinds thereof may be used in any proportion.

Examples of the polymerization solvent include water, an organic solvent, and a mixed solvent of water and the organic solvent.

Examples of the water include tap water, ion exchange water, pure water, and distilled water.

Examples of the organic solvent include alcohols such as methanol, ethanol, isopropyl alcohol, propyl alcohol, and butanol; ketones such as acetone and ethyl isobutyl ketone; ethylene glycols such as ethylene glycol and ethylene glycol methyl ether; propylene glycols such as propylene glycol, propylene glycol methyl ether, propylene glycol ethyl ether, propylene glycol butyl ether, and propylene glycol propyl ether; amides such as N,N-dimethylformamide and N,N-dimethylacetamide; and pyrrolidones such as N-methylpyrrolidone and N-ethylpyrrolidone.

As the polymerization solvent, water or a mixed solvent of water and the organic solvent is preferable.

Furthermore, when a mixed solvent of water and the organic solvent is used as the polymerization solvent, the mass ratio (water/organic solvent) is preferably 1/100 to 100/1 and more preferably 2/100 to 100/2.

The oxidizing agent is not limited as long as it is an oxidizing agent having a standard electrode potential of 0.6 V or more, and examples thereof include peroxodisulfates such as peroxodisulfuric acid, ammonium peroxodisulfate, sodium peroxodisulfate, and potassium peroxodisulfate; and hydrogen peroxide.

These oxidizing agents may be used alone or a mixture of two or more kinds thereof may be used in any proportion.

In addition to the polymerization solvent and the oxidizing agent, the raw material monomers may be polymerized in the presence of a basic reaction aid in the polymerization step.

Examples of the basic reaction aid include inorganic bases such as sodium hydroxide, potassium hydroxide, and lithium hydroxide; ammonia; fatty amines such as methylamine, dimethylamine, trimethylamine, ethylamine, diethylamine, triethylamine, ethyl methylamine, ethyl dimethylamine, and diethyl methylamine; cyclic saturated amines; and cyclic unsaturated amines such as pyridine, α-picoline, β-picoline, γ-picoline, and quinoline.

Among these, the inorganic bases, the fatty amines, and the cyclic unsaturated amines are preferable, and the cyclic unsaturated amines are more preferable.

Any one of these basic reaction aids may be used alone, or a mixture of two or more kinds thereof may be used in any proportion.

Examples of the polymerization method include a method in which a raw material monomer solution is added dropwise to an oxidizing agent solution, a method in which an oxidizing agent solution is added dropwise to a raw material monomer solution, and a method in which a raw material monomer solution and an oxidizing agent solution are simultaneously added dropwise to a reaction container. The raw material monomer solution may include a basic reaction aid as necessary.

As a solvent for the oxidizing agent solution and the raw material monomer solution, the above-described polymerization solvent can be used.

A reaction temperature of the polymerization reaction is preferably 50°C or lower, more preferably -15 to 30°C, and still more preferably -10 to 20°C. When the reaction temperature of the polymerization reaction is 50°C or lower, in particular, 30°C or lower, a progress of side reactions and a decrease in conductivity due to a change in the redox structure of the main chain of the generated conductive polymer (A) can be suppressed. When the reaction temperature of the polymerization reaction is -15°C or higher, a sufficient reaction rate can be maintained and the reaction time can be shortened.

Through the polymerization step, the conductive polymer (A) which is the reaction product is obtained in a state of being dissolved or precipitated in the polymerization solvent.

When the reaction product is dissolved in the polymerization solvent, the polymerization solvent is distilled off to obtain a reaction product.

When the reaction product is precipitated in the polymerization solvent, the polymerization solvent is filtered out by a filter such as a centrifuge to obtain a reaction product.

In the reaction products, low-molecular-weight components such as unreacted raw material monomers, oligomers accompanying the resulting side reactions, acidic substances (free acid groups eliminated from the conductive polymer (A), sulfate ions which are decomposition products of oxidizing agents, and the like), and basic substances (basic reaction aids, ammonium ions which are decomposition products of oxidizing agents, and the like) may be included. These low-molecular-weight components are impurities and cause the conductivity to be interfered.

Thus, it is preferable to purify the reaction product to remove the low-molecular-weight components.

### <<Purification Step>>

The purification step is a step of purifying the reaction product obtained in the polymerization step.

As a method for purifying the reaction product, any of methods such as a washing method using a washing solvent, a membrane filtration method, an ion exchange method, removal of impurities by a heat treatment, and neutralization precipitation can be used. Among these, the washing method and the ion exchange method are effective from the viewpoint that the conductive polymer (A) with a high purity can be easily obtained. In particular, from the viewpoint that raw material monomers, oligomers, acidic substances, and the like can be efficiently removed, the washing method is preferable. From the viewpoint that basic substances present in a state of forming a salt with the acidic group of the conductive polymer (A), and the like can be efficiently removed, the ion exchange method is preferable. In the purification step, a combination of the washing method and the ion exchange method may be used.

Examples of washing solvents include alcohols such as methanol, ethanol, n-propanol, isopropanol, n-butanol, isobutanol, 2-butanol, 3-butanol, t-butanol, 1-pentanol, 3-methyl-1-butanol, 2-pentanol, n-hexanol, 4-methyl-2-pentanol, 2-ethylbutynol, benzyl alcohol, furfuryl alcohol, and tetrahydrofurfuryl alcohol; polyhydric alcohol derivatives such as ethylene glycol monomethyl ether, ethylene glycol monoethyl ether, methoxy methoxyethanol, propylene glycol monoethyl ether, and glyceryl monoacetate; acetone, acetonitrile, N,N-dimethylformamide, N-methylpyrrolidone, and dimethyl sulfoxide. Among these, methanol, ethanol, isopropanol, acetone, and acetonitrile are effective.

When the reaction product after washing, that is, the conductive polymer (A) after washing is dried, the conductive polymer (A) in a solid form can be obtained.

Examples of ion exchange methods include column type or batch type treatments using ion exchange resins such as cation exchange resins and anion exchange resins; and electrodialysis methods.

Furthermore, when the reaction product is dissolved in the polymerization solvent, the contact may be performed with the ion exchange resin as it is in the dissolved state. When the concentration of the reaction product is high, dilution may be performed with an aqueous medium.

When the reaction product is precipitated in the polymerization solvent, it is preferable that a polymer solution, in which the reaction product is dissolved in an aqueous medium to the desired solid content concentration after filtration of the polymerization solvent, is prepared and brought into contact with the ion exchange resin.

When the reaction product after washing is subjected to an ion exchange treatment, it is preferable that a polymer solution, in which the reaction product after washing is dissolved in an aqueous medium to the desired solid content concentration, is prepared and brought into contact with the ion exchange resin.

Examples of the aqueous medium include the same ones as those of the solvent (D) which will be described later.

The concentration of the conductive polymer (A) in the polymer solution is preferably 0.1% to 20% by mass, and more preferably 0.1% to 10% by mass from the viewpoint of industrial efficiency and purification efficiency.

For an ion exchange method using an ion exchange resin, the amount of the sample solution with respect to the ion exchange resin is preferably up to 10 times the volume, and more preferably up to 5 times the volume with respect to the ion exchange resin, for example, for a polymer solution with a solid content concentration of 5% by mass. Examples of the cation exchange resin include "Amberlite IR-120B" manufactured by Organo Corporation. Examples of the anion exchange resin include "Amberlite IRA410" manufactured by Organo Corporation.

The conductive polymer (A) after the ion exchange treatment is in a state of being dissolved in the polymerization solvent or aqueous medium. Accordingly, when all of the polymerization solvent or aqueous medium is removed by an evaporator or the like, the solid conductive polymer (A) can be obtained, but the conductive polymer (A) may be used for the production of conductive compositions in a state of being dissolved in the polymerization solvent or aqueous medium.

### <Basic Compound (B)>

In a certain aspect, it is preferable that the conductive composition includes a basic compound (B).

When the conductive polymer (A) has an acidic group, it is considered that when the conductive composition includes the basic compound (B), the basic compound (B) efficiently acts on the acidic group in the conductive polymer (A), making it possible to enhance the stability of the conductive polymer (A). Here, the efficient action on the acidic group in the conductive polymer (A) means that the conductive polymer (A) can be stably neutralized and a stable salt is formed between the acidic group in the conductive polymer (A) and the basic compound (B). That is, the conductive polymer (A) is neutralized with the basic compound (B). As a result, the instability of the acidic group of the conductive polymer (A) due to hydrolysis in the conductive composition can be suppressed and the acidic group is less likely to be eliminated. In addition, by forming a stable salt between the acidic group of the conductive polymer (A) and the basic compound (B), the decomposition of the conductive polymer (A) itself can also be suppressed. Moreover, the basic compound (B) also forms a stable salt with a decomposition product (for example, a sulfate ion) of the oxidizing agent used for the production of the conductive polymer (A). Therefore, the generation of the acidic group derived from the conductive polymer (A) and the sulfate ion derived from the oxidizing agent in the conductive composition can be suppressed. In addition, the contained amount of the decomposition product of the conductive polymer (A) in the conductive composition can also be reduced.

Therefore, particularly in a pattern forming method by ionizing radiations using a chemical amplification type resist, a migration from the conductive film such as an acidic substance to the resist layer side is suppressed and the effect of a reduction in the film thickness of the resist layer and the like can be suppressed.

The basic compound (B) is not particularly limited as long as it is a compound having basicity, and examples thereof include a quaternary ammonium compound (b-1), a basic compound (b-2), a basic compound (b-3), and a basic compound (b-4).

Quaternary ammonium compound (b-1): A quaternary ammonium compound in which at least one of the four substituents bonded to the nitrogen atom is a hydrocarbon group with one or more carbon atoms.

Basic compound (b-2): A basic compound having one or more nitrogen atoms (provided that the quaternary ammonium compound (b-1) and the basic compound (b-3) are excluded).

Basic compound (b-3): A basic compound having a basic group and two or more hydroxy groups in the same molecule and a melting point of 30°C or higher.

Basic compound (b-4): An inorganic base.

In the quaternary ammonium compound (b-1), the nitrogen atom to which the four substituents are bonded is the nitrogen atom of the quaternary ammonium ion.

Examples of the hydrocarbon group bonded to the nitrogen atom of the quaternary ammonium ion in the quaternary ammonium compound (b-1) include an alkyl group, an aralkyl group, and an aryl group.

Examples of the quaternary ammonium compound (b-1) include tetramethylammonium hydroxide, tetraethylammonium hydroxide, tetrapropylammonium hydroxide, tetrabutylammonium hydroxide, tetrapentylammonium hydroxide, tetrahexylammonium hydroxide, and benzyltrimethylanunonium hydroxide.

Examples of the basic compound (b-2) include ammonia, pyridine, 4-dimethylaminopyridine, 4-dimethylaminomethylpyridine, 3,4-bis(dimethylamino)pyridine, picoline, triethylamine, 4-dimethylaminopyridine, 4-dimethylaminomethylpyridine, 3,4-bis(dimethylamino)pyridine, 1,5-diazabicyclo[4.3.0]-5-nonene (DBN), 1,8-diazabicyclo[5.4.0]-7-undecene (DBU), 1-(3-aminopropyl)-2-pyrrolidone, N-(3-aminopropyl)-ε-caprolactam, and derivatives thereof.

Examples of the basic group in the basic compound (b-3) include basic groups defined as Arrhenius bases, Brønsted bases, and Lewis bases. Specific examples thereof include ammonia. The hydroxy group may be in a state of -OH or may be in a state of being protected by a protecting group. Examples of the protecting group include an acetyl group; silyl groups such as a trimethylsilyl group and a t-butyldimethylsilyl group; acetal-type protecting groups such as a methoxymethyl group, an ethoxymethyl group, and a methoxyethoxymethyl group; a benzoyl group; and an alkoxide group.

Examples of the basic compound (b-3) include 2-amino-1,3-propanediol, tris(hydroxymethyl)aminomethane, 2-amino-2-methyl-1,3-propanediol, 2-amino-2-ethyl-1,3-propanediol, 3-[N-tris(hydroxymethyl)methylamino]-2-hydroxypropanesulfonic acid, and N-tris(hydroxymethyl)methyl-2-aminoethanesulfonic acid.

Examples of the basic compound (b-4) include sodium hydroxide, potassium hydroxide, and lithium hydroxide.

Any one of these basic compounds (B) may be used alone, or a mixture of two or more kinds thereof may be used in any proportion.

In a certain aspect, from the viewpoint of interaction with the conductive polymer (A), it is preferable that the conductive composition includes a quaternary ammonium compound (b-1). That is, the basic compound (B) preferably includes the quaternary ammonium compound (b-1).

### <Solvent (D)>

The solvent (D) is not particularly limited as long as it is a solvent capable of dissolving at least the conductive polymer (A) and the surfactant (C), and has the effect of the present invention, and examples thereof include water, an organic solvent, and a mixed solvent of water and the organic solvent.

Examples of the water include water among the polymerization solvents exemplified above in the description of the method for producing the conductive polymer (A).

Examples of the organic solvent include the organic solvent among the polymerization solvents exemplified above in the description of the method for producing the conductive polymer (A), and the good solvent exemplified above in the description of the surfactant (C). When the mixed solvent of water and the organic solvent is used as the solvent (D), a mass ratio of these solvents (water/the organic solvent) is preferably 1/100 to 100/1, and more preferably 2/100 to 100/2.

### (Optional Components)

Examples of the optional components include polymer compounds (provided that the conductive polymer (A), the basic compound (B), and the surfactant (C) are excluded), and an additive.

Examples of the polymer compounds include polyvinyl alcohol derivatives such as polyvinyl formal and polyvinyl butyral and modified products thereof, starch and modified products thereof (starch oxide, phosphate esterified starch, cationized starch, and the like), cellulose derivatives (carboxymethyl cellulose, methylcellulose, hydroxypropyl cellulose, hydroxyethylcellulose, salts thereof, and the like), polyacrylamides such as polyacrylamide, poly(N-t-butylacrylamide), and polyacrylamidomethylpropanesulfonic acid, polyvinylpyrrolidone, polyacrylic acids (salts), polyethylene glycol, water soluble alkyd resins, water soluble melamine resins, water soluble urea resins, water soluble phenolic resins, water soluble epoxy resins, water soluble polybutadiene resins, water soluble acrylic resins, water soluble urethane resins, water soluble acrylstyrene copolymer resins, water soluble vinyl acetate acrylic copolymer resins, water soluble polyester resins, water soluble styrene-maleic acid copolymerized resins, water soluble fluororesins, and copolymers thereof.

Examples of the additive include a pigment, a defoamer, an ultraviolet absorber, an antioxidant, a heat resistance improver, a leveling agent, an anti-sagging agent, a matting agent, and a preservative.

### (Contained Amount)

The contained amount of the conductive polymer (A) is preferably 0.1% by mass or more, more preferably 0.2% by mass or more, and still more preferably 0.5% by mass or more, and is preferably 5% by mass or less, more preferably 3% by mass or less, and still more preferably 2% by mass or less, with respect to the total mass of the conductive composition. The lower limit and the upper limit of the contained amount of the conductive polymer (A) can be optionally combined, and for example, the contained amount of the conductive polymer (A) is preferably 0.1% to 5% by mass, more preferably 0.2% to 3% by mass, and still more preferably 0.5% to 2% by mass.

In addition, the contained amount of the conductive polymer (A) is preferably 50% to 99.9% by mass, more preferably 80% to 99.9% by mass, and still more preferably 95% to 99.9% by mass with respect to the total mass of the solid content of the conductive composition. Furthermore, the solid content of the conductive composition is the remainder of the conductive composition excluding the solvent (D).

When the contained amount of the conductive polymer (A) is within the range, there is an excellent balance between the coating properties of the conductive composition and the conductivity of the conductive film formed from the conductive composition.

The contained amount of the basic compound (B) is preferably 5 to 80 parts by mass, more preferably 5 to 50 parts by mass, and still more preferably 5 to 30 parts by mass with respect to 100 parts by mass of the conductive polymer (A).

When the contained amount of the basic compound (B) is equal to or more than the lower limit value, even though the conductive polymer (A) includes an acidic group, the acidic group of the conductive polymer (A) can sufficiently form a salt with the oxidizing agent, and the acidic group is less likely to be eliminated. In addition, the basic compound (B) also sufficiently forms a salt with a decomposition product of the oxidizing agent. Thus, the migration of the acidic group and the decomposition product of the oxidizing agent to the resist layer is suppressed and a reduction in the film thickness of the resist layer can be suppressed. When the contained amount of the basic compound (B) is equal to or less than the upper limit value, the contained amount of the conductive polymer (A) in the conductive composition can be sufficiently secured, and thus, a conductive film having favorable conductivity can be formed. In addition, when the conductive polymer (A) includes an acidic group, the acidic group is moderately present in a free state without forming salts with the basic compound (B) in the conductive film, and thus, the conductivity can be favorably maintained.

In particular, when the conductive polymer (A) has an acidic group or a salt of the acidic group, the contained amount of the basic compound (B) is preferably 0.1 to 1 molar equivalent, and more preferably 0.1 to 0.9 molar equivalents with respect to 1 mol of the units having an acidic group among the units constituting the conductive polymer (A) from the viewpoint of further improving the coating properties of the conductive composition. Furthermore, the contained amount of the acidic group is particularly preferably 0.25 to 0.85 molar equivalents from the viewpoint of having excellent retention of the performance as a conductive film and making it possible to further stabilize the acidic group in the conductive polymer (A).

The contained amount of the surfactant (C) is preferably 5 to 200 parts by mass, and more preferably 10 to 100 parts by mass with respect to 100 parts by mass of the conductive polymer (A). When the contained amount of the surfactant (C) is within the range, the coating properties of the conductive composition onto the resist layer is further improved.

The contained amount of the solvent (D) is preferably 1% to 99% by mass, more preferably 10% to 99% by mass, and still more preferably 50% to 99% by mass with respect to the total mass of the conductive composition. When the contained amount of the solvent (D) is within the range, the coating properties are further improved.

Furthermore, when the conductive polymer (A) is used in a state of being dispersed or dissolved in an aqueous medium by purification or the like (in which the conductive polymer (A) in this state is hereinafter also referred to as a "conductive polymer solution"), the aqueous medium derived from the conductive polymer solution is also included in the contained amount of the solvent (D) in the conductive composition.

In addition, the total amount of all the components constituting the conductive composition does not exceed 100% by mass.

### <Method for Producing Conductive Composition>

An embodiment of the present invention is a method for producing a conductive composition.

The method for producing a conductive composition of the present embodiment includes a step of producing a water soluble composition (C) by the above-described method for producing a surfactant of the present invention (hereinafter also referred to as a "water soluble composition production step"), and a step of mixing the water soluble composition obtained in the water soluble composition production step with the conductive polymer (A) (hereinafter also referred to as a "mixing step").

In the water soluble composition production step, for example, when the water soluble composition (C) is purified by a reprecipitation method, the water soluble composition (C) is obtained in a state of wet powder, as described above. The wet powder may be subjected to the mixing step after being dried, or may be subjected to the mixing step as it is in the wet powder state.

In the mixing step, the above-described conductive polymer (A) and the water soluble composition (C) are mixed.

In the mixing step, it is preferable to mix the conductive polymer (A) and the water soluble composition (C) with, as necessary, one or more of the basic compound (B), the solvent (D), and the optional components.

For example, since the conductive polymer (A) after washing, produced by the above-described method for producing the conductive polymer (A), is in a solid form, the conductive composition may be obtained by mixing the conductive polymer (A) and the water soluble composition (C) in a solid form and the solvent (D), and as necessary, one or more of the basic compound (B), the solvent (D), and the optional components.

Furthermore, when the conductive polymer (A) further purified by the ion exchange method after washing is used, the conductive polymer (A) after the ion exchange treatment is obtained in a state of the conductive polymer solution as described above. Therefore, the conductive polymer solution and the water soluble composition (C) may be mixed to obtain a conductive composition, one or more of the basic compound (B) and the optional component may be further added thereto as necessary, or the composition may be further diluted with the solvent (D).

### <Action Effect>

Since the conductive composition of the present embodiment described above includes the above-described water soluble composition of the present invention, the conductive composition has favorable coating properties and is less likely to generate foreign matter during formation of a coating film.

### <Use>

The conductive composition of the present embodiment is suitable for antistatic purposes during the charged particle beam drawing. Specifically, the conductive composition of the present embodiment is applied to the surface of a resist layer in a pattern forming method using charged particle beams with a chemical amplification type resist or a non-chemical amplification type resist to form a conductive film. The conductive film thus formed serves as an antistatic film of the resist layer.

In addition to those above, for example, it is also possible to use the conductive composition of the present embodiment as a material for capacitors, transparent electrodes, semiconductors, and the like.

### [Conductive film and Method for Producing Same]

An embodiment of a conductive film formed of the conductive composition of the present invention and a method for producing the same will be described.

The conductive film of the present embodiment is a film formed of the above-described conductive composition of the present invention, and includes the above-described conductive polymer (A) and the water soluble composition (C) of the present invention.

The conductive film may further include the above-described basic compound (B) in addition to the conductive polymer (A) and the water soluble composition (C). In addition, the conductive film may further include the above-described optional components in addition to the conductive polymer (A), the basic compound (B), and the water soluble composition (C).

The method for producing a conductive film of the present embodiment includes a film formation step described below.

The method for producing a conductive film of the present embodiment may further include a base material preparation step and a resist layer formation step described below before the film formation step.

### <Base Material Preparation Step>

Examples of the base material include polyester resins such as polyethylene terephthalate (PET) and polybutylene terephthalate (PBT), polyolefin resins typified by polyethylene and polypropylene, vinyl chloride, nylon, polystyrene, polycarbonate, epoxy resins, fluororesins, polysulfone, polyimide, silicone resins, polyurethane, phenol resins, and molded articles and films of these polymer compounds such as synthetic papers; paper, iron, glass, quartz glass, various wafers, various mask blanks, aluminum, copper, zinc, nickel, and stainless steel; base materials whose surfaces are coated with various paints, photosensitive resins, or the like.

The shape of the base material is not particularly limited and may be plate-like or a non-plate-like shape.

### <Resist Layer Formation Step>

The resist layer formation step is a step of forming a resist layer on a base material.

Furthermore, in the present specification, the base material on which the resist layer is formed is also referred to as a "base material with a resist layer".

The resist layer is obtained by applying a resist onto a base material.

Examples of the resist include a positive tone resist and a negative tone resist.

The positive tone resist is not particularly limited as long as it is sensitive to ionizing radiations and a known resist can be used. Typical examples thereof include a chemical amplification type resist which includes an acid generator that generates an acid upon irradiation with ionizing radiations and a polymer that includes a constitutional unit having an acid-decomposable group.

The negative tone resist is not particularly limited as long as it is sensitive to ionizing radiations and a known resist can be used. Typical examples thereof include a chemical amplification type resist which includes an acid generator that generates an acid upon irradiation with ionizing radiations, a polymer that is soluble in a developer, and a crosslinking agent.

The resist layer can be formed by a known method. For example, an organic solvent solution of a positive tone or negative tone resist is applied onto one surface of the base material, and subjected to heating (pre-baking) as necessary to form a positive tone or negative tone resist layer. The method for applying the resist is the same as the method for applying a conductive composition which will be described later.

The film thickness of the resist layer is not particularly limited, but is, for example, preferably 50 to 200 nm, and more preferably 70 to 150 nm.

### <Film Formation Step>

The film formation step is a step of forming a conductive film on a base material.

The film formation step is a step of forming a conductive film.

The conductive film is obtained by applying the conductive composition onto a base material and drying the applied composition. A heat treatment may be performed, as necessary, after drying.

When a resist layer is formed on a base material, a conductive film is formed on the resist layer of the base material with the resist layer.

When the resist layer is not formed on the base material, the conductive film is formed directly on a surface of the base material.

The conductive film is preferably a conductive film formed on a base material or a conductive film formed on a resist layer of a base material with a resist layer.

Examples of the method for applying the conductive composition include methods such as a spin coating method, a spray coating method, a dip coating method, a roll coating method, a gravure coating method, a reverse coating method, a roll brushing method, an air knife coating method, and a curtain coating method.

A drying temperature of lower than 40°C is preferable. The drying time is preferably less than one hour.

For the heat treatment temperature, from the viewpoint of conductivity, a temperature range of 40°C to 250°C is preferable and a temperature range of 60°C to 200°C is more preferable. From the viewpoint of stability, the treatment time is preferably within one hour and more preferably within 30 minutes.

The film thickness of the conductive film is preferably 1 to 100 nm, more preferably 5 to 50 nm, and still more preferably 5 to 30 nm.

### <Action Effect>

Since the conductive film of the present embodiment described above is formed of the conductive composition of the present invention, the generation of foreign matter is suppressed.

### <Use>

The method for producing a conductive film of the present embodiment can be applied to, for example, a method for producing a mask blank with a conductive film, a method for producing a mask, and a method for producing a semiconductor device.

### [Method for Producing Mask Blank with Conductive Film]

An embodiment of a method for producing a mask blank with a conductive film will be described.

The method for producing a mask blank with a conductive film of the present embodiment includes a step of forming a conductive film on a mask blank using the above-described method for producing a conductive film of the present invention.

Specifically, the method is as follows.

The mask blank is used as a base material and a conductive film is formed on the mask blank, thereby obtaining a mask blank with a conductive film. A method for forming the conductive film is the same as the above-described film formation step.

Furthermore, it is preferable that a resist layer is formed on the mask blank before the conductive film is formed on the mask blank. That is, it is preferable that the conductive film is formed on the resist layer of the mask blank on which the resist layer is formed. A method for forming the resist layer is the same as the above-described resist layer formation step.

As the mask blank, a known mask blank can be used and a mask blank corresponding to the configuration of the target mask may be used. Specific examples of the mask blank include those provided with a light transmitting substrate and a thin film provided on the light transmitting substrate.

Examples of the light transmitting substrate include soda-lime glass, low expansion glass, and quartz glass.

Examples of the thin film include a light shielding film, an antireflection film, a phase shift film, and an antioxidant film. The thin film may have a monolayer structure or a multilayer structure.

### [Method for Producing Mask]

An embodiment of a method for producing a mask will be described.

The method for producing a mask of the present embodiment includes a step of producing a mask blank with a conductive film and a step of producing a mask using the obtained mask blank with a conductive film.

Specifically, the method is as follows.

First, a mask blank with a conductive film is produced. For example, a conductive film may be formed on a mask blank by the above-described method for producing a conductive film to produce the mask blank with a conductive film, or the above-described method for producing a mask blank with a conductive film may be used to produce the mask blank with a conductive film.

Furthermore, it is preferable that a resist layer is formed on the mask blank before the conductive film is formed on the mask blank. That is, it is preferable to produce a mask using a mask blank with a conductive film, in which the resist layer and the conductive film are formed in this order on the mask blank.

Next, a mask is produced using the mask blank with a conductive film. The step of producing a mask includes, for example, an ionizing radiation irradiation step, a water washing and development step, an etching step, and a resist layer removal step, each shown below.

The ionizing radiation irradiation step is a step of irradiating the resist layer of the mask blank with a conductive film with ionizing radiations in a patterned manner. Specifically, the mask blank with a conductive film is irradiated with ionizing radiations from the side of the conductive film.

Examples of the ionizing radiations include charged particle beams such as electron beams and ion beams; and electromagnetic waves such as X rays and gamma rays.

In the ionizing radiation irradiation step, a latent image is formed in the resist layer between the mask blank and the conductive film. When the resist layer consists of a chemical amplification type resist, an acid is generated from the acid generator in the part irradiated with ionizing radiations.

At this time, since the conductive film is provided on the surface of the resist layer, grounding can be taken from the conductive film and the entire mask blank with the conductive film can be prevented from being charged. Therefore, a shift of the positions of electrons incident on the resist layer due to an effect of the charging can be suppressed and a latent image corresponding to the target resist pattern can be formed with a high accuracy.

The water washing and development step is a step of removing the conductive film by subjecting the resist layer to water washing and development to form a resist pattern.

Since the conductive film is water soluble, the conductive film is dissolved and removed when the water washing is performed.

The water washing represents bringing into contact with an aqueous liquid. Examples of the aqueous liquid include simple water, water including at least one of a base and a basic salt, water including an acid, and a mixture of water and a water soluble organic solvent.

When the positive tone resist layer is developed, the part of the positive tone resist layer which has been irradiated with ionizing radiations is dissolved and removed, and a resist pattern consisting of a part of the positive tone resist layer which has not been irradiated with ionizing radiations is formed.

On the other hand, when the negative tone resist layer is developed, unlike the case of the positive tone resist layer, the part which has not been irradiated with ionizing radiations is dissolved and removed, and a resist pattern consisting of the part of the negative tone resist layer which has been irradiated with ionizing radiations is formed.

The water washing and development step can be performed, for example, by the following method (α) or (β).

Method (α): A resist layer is developed while removing the conductive film, using an alkaline developing solution for water washing.

Method (β): Only the conductive film is removed by water washing and then the resist layer is developed with a developing solution.

The alkaline developing solution (aqueous alkaline solution) used in the method (α) is not particularly limited, and any known alkaline developing solution can be used. Examples thereof include an aqueous tetramethylammonium hydroxide solution.

The water washing in the method (β) can be performed using an aqueous liquid not corresponding to a developing solution, for example, water. The development can be performed using an alkaline developing solution in the same manner as in the method (α).

In addition, after the water washing and development step, the substrate may be subjected to a rinsing treatment with pure water or the like, as necessary.

After the water washing and development step or after the rinsing treatment, the substrate on which the resist pattern is formed may be heated (post-baked), as necessary.

The etching step is a step of etching a thin film of a mask blank, using a resist pattern as a mask. By the etching, a thin film of a part which is not masked by the resist pattern is removed (that is, an exposed part).

As the etching method, a known method can be adopted, and examples thereof include dry etching and wet etching.

The resist removal step is a step of removing a resist pattern remaining on the mask blank after the etching. By removing the resist pattern, a mask (photomask) on which a pattern is formed is obtained.

A known peeling agent can be used for removing the resist pattern.

### [Method for Producing Semiconductor Device]

An embodiment of a method for producing a semiconductor device will be described.

The method for producing a semiconductor device of the present embodiment includes a step of forming a conductive film on a base material using the above-described method for producing a conductive film to obtain a laminate and a step of producing a semiconductor device using the laminate.

Specifically, the method is as follows.

First, a conductive film is formed on a base material to obtain a laminate. A method for forming the conductive film is the same as the above-described film formation step.

Furthermore, it is preferable that a resist layer is formed on a base material before the conductive film is formed on the base material. That is, it is preferable that a conductive film is formed on the resist layer of the base material with a resist layer using the base material with a resist layer, and a semiconductor device is produced using a laminate in which the resist layer and the conductive film are formed in this order on the base material. A method for forming the resist layer is the same as the above-described resist layer formation step.

As the base material, a known base material used as a base material for a semiconductor device can be used. Examples thereof include a silicon wafer.

Next, a semiconductor device is produced using the laminate. The step of producing a semiconductor device includes, for example, an ionizing radiation irradiation step shown below. In addition, the method further includes a water washing step, a development step, an etching step, and a resist layer removal step, each shown below, as necessary.

The ionizing radiation irradiation step is a step of irradiating the laminate with ionizing radiations such as electron beams and ion beams to form a wiring pattern.

At this time, since a conductive film is provided on a surface of the base material or the resist layer, it is possible to ground the entire laminate from the conductive film and to prevent the entire laminate from being charged. Therefore, a shift of the positions of electrons incident on the base material or the resist layer due to an effect of the charging can be suppressed and a latent image corresponding to the transferred pattern can be formed with a high accuracy.

The water washing step and the development step are the same as the water washing and development step in the above-described method for producing a mask.

The water washing step and the development step may be performed at the same time (the method (α)), or the development step may be performed after the water washing step (the method (β)).

The etching step is the same as the etching step in the above-described method for producing a mask.

The resist layer removal step is the same as the resist layer removal step of the above-described method for producing a mask.

### Examples

Hereinafter, the present invention will be described in more detail with reference to Examples and Comparative Examples, but the present invention is not limited thereto.

### [Production of Conductive Polymer (A)]

100 mmol of pyridine and 100 mL of water were added to 100 mmol of 2-aminoanisole-4-sulfonic acid to obtain a monomer solution.

An aqueous solution of 100 mmol of ammonium peroxodisulfate (oxidizing agent solution) was added dropwise to the obtained monomer solution at 10°C. After finishing the dropwise addition, the mixture was further stirred at 25°C for 15 hours, then the temperature was increased to 35°C, and the mixture was stirred for another two hours to obtain a reaction solution in which the reaction product was precipitated (polymerization step).

The obtained reaction solution was filtered through a centrifugal filter, the precipitate (reaction product) was recovered, and the reaction product was washed with 1 L of methanol and then dried. 20 g of the reaction product after drying was dissolved in 980 g of pure water and 1,000 g of conductive polymer solution (A1-1) with a solid content concentration of 2% by mass was obtained.

A column was filled with 500 mL of a cation exchange resin ("Amberlite IR-120B", manufactured by Organo Corporation), which was washed with ultrapure water.

1,000 g of the conductive polymer solution (A1-1) was passed through this column at a rate of 50 mL/minute (SV = 6) to obtain 900 g of a conductive polymer solution (A1-2) from which basic substances and the like had been removed.

Next, 500 mL of an anion exchange resin ("Amberlite IRA410", manufactured by Organo Corporation) washed with ultrapure water was filled into the column.

900 g of the conductive polymer solution (A1-2) was passed through this column at a rate of 50 mL/minute (SV = 6) to obtain 800 g of a conductive polymer solution (Al-3) from which basic substances and the like had been removed.

As a result of measuring the heating residue of this conductive polymer solution (Al-3), the heating residue was 2.0% by mass. That is, the solid content concentration of the conductive polymer solution (A1-3) is 2.0% by mass.

Furthermore, one sverdrup (SV) is defined as 1 × 10⁶ m³/s (1 GL/s).

### [Measurement and Evaluation methods]

### <Quantification of Component (X)>

The contained amount of the component (X) in the water soluble composition (C) was quantified by the following measurement method.

### (Measurement Method)

### <<Creation of Calibration Curve>>

n-Dodecyl mercaptan, which is a chain transfer agent used in the production of the water soluble composition (C), was dissolved in a mixed solvent at a volume ratio of water to methanol (water/methanol) of 2/8 to give concentrations of each 500 mg/L, 100 mg/L, 50 mg/L, and 10 mg/L, thereby preparing each of solutions (III-1) to (III-4).

Separately, ethyl n-caproate was dissolved in methanol to give a concentration of 5 mg/L, thereby preparing a solution (1).

Each of the solutions (III-1) to (III-4) was mixed with the solution (I) at a volume ratio (solution (I)/solution (III)) of 1/10 to prepare each of samples (S2) to (S5), and the samples were subjected to gas chromatography analysis under the following conditions.

After injecting each of the samples (S2) to (S5) into gas chromatography, in 35-minute analysis in which a time point at which the column oven was heated to 50°C was set as 0 minutes and the temperature was raised to 300°C at 10°C/minute and maintained at 300°C for 10 minutes, each peak area obtained between 25 minutes and 35 minutes was determined, and a calibration curve showing a relationship between the concentration ratio of n-dodecyl mercaptan to ethyl n-caproate as an internal standard substance and the peak area ratio was created.

### <<Quantification of Component (X)>>

Ethyl n-caproate was dissolved in methanol to give a concentration of 5 mg/L, thereby preparing a solution (I).

Separately, the water soluble composition (C) was dissolved in a mixed solvent at a volume ratio of water to methanol (water/methanol) of 2/8 to give a concentration of 0.2% by mass, thereby preparing a solution (II).

The solution (I) was mixed with the solution (II) at a volume ratio (solution (I)/solution (II)) of 1/10 to prepare a sample (S1), and the sample was subjected to gas chromatography analysis under the following conditions.

After injecting the sample (S1) into gas chromatography, in 35-minute analysis in which a time point at which the column oven was heated to 50°C was set as 0 minutes and the temperature was raised to 300°C at 10°C/minute and maintained at 300°C for 10 minutes, a peak area obtained between 25 minutes and 35 minutes was quantified by comparison with a calibration curve created in advance to perform quantification, and the value was defined as a contained amount (concentration) of the component (X) in the water soluble composition (C).

Here, the quantification was performed by converting the relative value to an absolute value with respect to ethyl n-caproate as an internal standard substance.

### <Conditions for Gas Chromatography Analysis>

· Apparatus: 7890 Gas Chromatograph (manufactured by Agilent Technologies, Inc.).
· Column: DB-5 (30 m × 0.53 mmφ × 1.5 µm) column No. E11 (manufactured by Agilent Technologies, Inc.).
· Injection port temperature: 200°C.
· Detector: FID.
· Detector temperature: 300°C.
· Injection method: Splitless.
· Carrier gas: He.
· Pressure: 35 kPa (constant pressure).
· Initial flow rate of column: 7 mL/min.
· Injection volume: 1 µL.

### <Measurement of Molecular Weight of Water Soluble Polymer (B)>

A 0.1% by mass aqueous solution of the water soluble polymer (B) was filtered through a membrane filter having a pore size of 0.45 µm to adjust the sample. The GPC measurement of the sample was performed under the following conditions to measure the mass-average molecular weight of the water soluble polymer (B).

### <<Conditions for GPC Measurement>>

· Measuring machine: TOSOH GPC-8020 (manufactured by Tosoh Corporation)
· Eluent: 0.2 M NaNO₃-DIW/acetonitrile = 80/20 (v/v)
· Column temperature: 30°C
· Calibration curve: Created using EasiVial^{™} polyethylene glycol/oxide (manufactured by Polymer Labs Inc.)

### <Evaluation of Appearance>

The appearance of an aqueous solution prepared by dissolving the water soluble composition (C) in water to give a concentration of 1.0% by mass or 0.5% by mass was visually observed, and the appearance was evaluated in accordance with the following evaluation standards. A is determined to be acceptable.
A: No turbidity is observed even in the aqueous solution having a concentration of 1.0% by mass.
B: Turbidity occurs in the aqueous solution having a concentration of 1.0% by mass, but no turbidity was observed in the aqueous solution having a concentration of 0.5% by mass.
C: Turbidity occurs in the aqueous solution having a concentration of 0.5% by mass.

### <Evaluation of Conductivity>

A conductive composition was applied onto an 8-inch silicon wafer as a base material by spin coating (1,000 rpm × 180 sec), and heated on a hot plate at 80°C × 2 minutes to obtain a test piece in which a conductive film (film thickness: 20 nm) was formed on the base material. The surface resistivity [Ω/□] of the conductive film was measured by a two-terminal method (distance between electrodes: 20 mm) using Hiresta UX-MCP-HT800 (manufactured by NITTOSEIKO Analytech Co., Ltd.).

### <Measurement of pH>

The pH of the conductive composition at 25°C was measured using a pH meter (manufactured by HORIBA, Ltd., product name "F-55").

<Evaluation of Coating Properties>1 mL of the conductive composition was added dropwise onto an 8-inch silicon wafer as a base material, and applied using a spin coater. The coating state was visually observed, and the coating properties were determined in accordance with the following evaluation standards. A is determined to be acceptable.
A: The composition is applied on the wafer without unevenness.
B: The composition is applied on the wafer but unevenness is present.

### <Evaluation of Generation of Foreign Matter>

A conductive composition was applied onto an 8-inch silicon wafer as a base material by spin coating (1,000 rpm × 180 sec), and heated on a hot plate at 80°C × 2 minutes to obtain a test piece in which a conductive film (film thickness: 20 nm) was formed on the base material.

The obtained test piece was observed with a microscope (magnification: 1,000 times) using an SEM linked to an optical wafer appearance inspection device (manufactured by Hitachi High-Tech Corporation, product name "SR-7300"), and the number of foreign matter pieces generated per mm² of the conductive film (α) was measured. Furthermore, as the foreign matter, foreign matter having a longest diameter of 0.3 µm or more was measured.

In the same manner, the number (β) of foreign matter pieces having a longest diameter of 0.3 µm or more present per mm² of the base material was measured on an 8-inch silicon wafer in advance before the application of the conductive composition, the number (α - β) of foreign matter pieces derived from the conductive composition was determined, and the occurrence of foreign matters was evaluated in accordance with the following evaluation standard. A is determined to be acceptable.
AA: The number of foreign matter pieces derived from the conductive composition is 30 or less.
A: The number of foreign matter pieces derived from the conductive composition is 31 to 50.
B: The number of foreign matter pieces derived from the conductive composition is 51 to 100.
C: The number of foreign matter pieces derived from the conductive composition is 101 or more.

### [Example 1]

### <Production of Water Soluble Composition (C)>

55 g (0.49 mol) of N-vinylpyrrolidone, 3 g (15.60 mmol) of azobismethylbutyronitrile as a polymerization initiator, and 1 g (4.94 mmol) of n-dodecyl mercaptan as a chain transfer agent were dissolved under stirring in 100 mL of isopropyl alcohol to obtain a reaction solution.

Thereafter, the reaction solution was added dropwise at a rate of 1 mL/minute to 100 mL of isopropyl alcohol, which was heated in advance to 80°C, and dropwise polymerization was performed. Dropwise polymerization was performed while maintaining the temperature of the isopropyl alcohol at 80°C. After finishing the dropwise addition, the mixture was further aged at 80°C for 2 hours to complete polymerization (polymerization step).

Thereafter, the solution was allowed to cool and further concentrated under reduced pressure to obtain a water soluble polymer solution. As a result of measuring the heating residue of this water soluble polymer solution, the heating residue was 60.0% by mass. That is, the solid content concentration of the water soluble polymer solution is 60.0% by mass.

Next, 50 g of diethyl ether and 50 g of hexane were mixed as a poor solvent and stirred, and 10 g of the water soluble polymer solution was added dropwise to the poor solvent. After adding dropwise the total amount of the water soluble polymer solution, the solution was stirred for 30 minutes (reprecipitation step). Furthermore, the "Amount of poor solvent" in Table 1 is the amount (mixed amount) (parts by mass) of the poor solvent used in the reprecipitation step with respect to 100 parts by mass of the water soluble polymer solution, which is the good solvent solution.

Next, the precipitate generated in the poor solvent was recovered by decantation, and dried under reduced pressure at 70°C for 3 hours to obtain a powder of a water soluble composition (C1) including a water soluble polymer having interfacial performance.

The component (X) was quantified for the obtained water soluble composition (C1) and the appearance of the aqueous solution was evaluated. The results are shown in Table 1.

The mass-average molecular weight of the water soluble polymer was 1,200.

A 1.0% aqueous solution of the water soluble composition (C1) had a surface tension of 35.3.

### <Production of Conductive Composition>

30 parts by mass (0.6 parts by mass in terms of solid content) of the conductive polymer solution (A1-3), 0.3 parts by mass of tetrabutylammonium hydroxide as the basic compound (B), 0.1 parts by mass of the water soluble composition (C1), 5 parts by mass of isopropyl alcohol as the solvent (D), and 64.6 parts by mass of water were mixed to obtain a conductive composition.

The contained amounts of the conductive polymer (A), the basic compound (B), the water soluble composition (C), and the solvent (D) with respect to the total mass of the conductive composition are shown in Table 2. Furthermore, the contained amount of the conductive polymer (A) in Table 2 is an amount in terms of solid content (pure amount). In addition, the contained amount of water also includes the amount of water (29.4 parts by mass) derived from the conductive polymer solution (A1-3).

The pH of the obtained conductive composition was measured, and the conductivity, the coating properties, and the generation of foreign matter were evaluated. The results are shown in Table 2.

### [Example 2 and 3]

Water soluble compositions (C2) and (C3) were produced in the same manner as in Example 1, except that a poor solvent of the type shown in Table 1 was used. The component (X) was quantified for the obtained water soluble compositions (C2) and (C3), and the appearance of the aqueous solution was evaluated. The results are shown in Table 1.

In addition, a conductive composition was produced in the same manner as in Example 1, except that the obtained water soluble compositions (C2) and (C3) were used. The pH of the obtained conductive composition was measured, and the conductivity, the coating properties, and the generation of foreign matter were evaluated. The results are shown in Table 2.

A 1.0% aqueous solution of the water soluble composition (C2) had a surface tension of 35.1.

### [Example 4]

A water soluble polymer solution having a solid content concentration of 60.0% by mass was produced in the same manner as in Example 1, except that 3 g of dilauroyl peroxide was used instead of 3 g of azobismethylbutyronitrile as the polymerization initiator, and the same amount of ethyl acetate was used instead of isopropyl alcohol as the solvent. Next, a reprecipitation step was performed in the same manner as in Example 1 to produce a water soluble composition (C4), except that a poor solvent of the type shown in Table 1 was used. The component (X) was quantified for the obtained water soluble composition (C4) and the appearance of the aqueous solution was evaluated. The results are shown in Table 1.

In addition, a conductive composition was produced in the same manner as in Example 1, except that the obtained water soluble composition (C4) was used. The pH of the obtained conductive composition was measured, and the conductivity, the coating properties, and the generation of foreign matter were evaluated. The results are shown in Table 2. A 1.0% aqueous solution of the water soluble composition (C4) had a surface tension of 34.3.

### [Comparative Example 1]

A water soluble polymer solution having a solid content concentration of 60.0% by mass was produced in the same manner as in Example 1.

The obtained water soluble polymer solution was dried under reduced pressure at a temperature of 70°C for 3 hours to obtain a powder of a water soluble composition (C5) which is a water soluble polymer. The component (X) was quantified for the obtained water soluble composition (C5) and the appearance of the aqueous solution was evaluated. The results are shown in Table 1.

In addition, a conductive composition was produced in the same manner as in Example 1, except that the obtained water soluble composition (C5) was used. The pH of the obtained conductive composition was measured, and the conductivity, the coating properties, and the generation of foreign matter were evaluated. The results are shown in Table 2.

### [Comparative Example 2]

Water soluble composition (C6) was produced in the same manner as in Example 1, except that a poor solvent of the type shown in Table 1 was used. The component (X) was quantified for the obtained water soluble composition (C6) and the appearance of the aqueous solution was evaluated. The results are shown in Table 1.

In addition, a conductive composition was produced in the same manner as in Example 1, except that the obtained water soluble composition (C6) was used. The pH of the obtained conductive composition was measured, and the conductivity, the coating properties, and the generation of foreign matter were evaluated. The results are shown in Table 2.

A 1.0% aqueous solution of the water soluble composition (C6) had a surface tension of 33.3.

### [Comparative Examples 3 to 5]

A water soluble polymer solution having a solid content concentration of 60.0% by mass was produced in the same manner as in Example 1, except that 3 g of dilauroyl peroxide was used instead of 3 g of azobismethylbutyronitrile as the polymerization initiator, and the same amount of ethyl acetate was used instead of isopropyl alcohol as the solvent. Next, a reprecipitation step was performed in the same manner as in Example 1 to produce a water soluble compositions (C7) to (C9), except that a poor solvent of the type shown in Table 1 was used. The component (X) was quantified for the obtained water soluble compositions (C7) to (C9) and the appearance of the aqueous solution was evaluated. The results are shown in Table 1.

The mass-average molecular weight of the water soluble polymer was 2,200.

In addition, conductive compositions were produced in the same manner as in Example 1, except that the obtained water soluble compositions (C7) to (C9) were used. The pH of the obtained conductive composition was measured, and the conductivity, the coating properties, and the generation of foreign matter were evaluated. The results are shown in Table 2.

A 1.0% aqueous solution of the water soluble composition (C7) had a surface tension of 32.8.

A 1.0% aqueous solution of the water soluble composition (C8) had a surface tension of 33.5.

**[Table 1]**

| | | | Example | | | | Comparative Example | | | | |
|---|---|---|---|---|---|---|---|---|---|---|---|
| | | | 1 | 2 | 3 | 4 | 1 | 2 | 3 | 4 | 5 |
| Type of water soluble composition | | | C1 | C2 | C3 | C4 | C5 | C6 | C7 | C8 | C9 |
| Polymerization step | Vinyl monomer | | NVP | NVP | NVP | NVP | NVP | NVP | NVP | NVP | NVP |
| | Chain transfer agent | | nDM | nDM | nDM | nDM | nDM | nDM | nDM | nDM | nDM |
| | Polymerization initiator | | AMBN | AMBN | AMBN | LPO | AMBN | AMBN | AMBN | AMBN | LPO |
| | Solvent | | IPA | IPA | IPA | EtOAc | IPA | IPA | IPA | IPA | EtOAc |
| Reprecipitation step | Poor solvent | Type | DEE/HEX | DIPE/HEX | TBME/HEX | DIPE/HEX | Unpurified | HEX | DIPE | TBME | HEX |
| | | Proportion [% by mass] | 50/50 | 50/50 | 50/50 | 50/50 | - | 100 | 100 | 100 | 100 |
| | Concentration [% by mass] of water soluble polymer solution | | 60 | 60 | 60 | 60 | - | 60 | 60 | 60 | 60 |
| | Amount [parts by mass] of poor solvent | | 1000 | 1000 | 1000 | 1000 | - | 1000 | 1000 | 1000 | 1000 |
| Concentration [ppm by mass] of component (X) | | | 0.1 | 1.0 | 0.9 | 1.2 | 3.3 | 1.4 | 1.8 | 1.7 | 1.5 |
| Evaluation | Appearance | | A | A | A | A | C | B | C | C | B |

**[Table 2]**

| | | | Example | | | | Comparative Example | | | | |
|---|---|---|---|---|---|---|---|---|---|---|---|
| | | | 1 | 2 | 3 | 4 | 1 | 2 | 3 | 4 | 5 |
| Type of water soluble composition | | | C1 | C2 | C3 | C4 | C5 | C6 | C7 | C8 | C9 |
| Polymerization step | Vinyl monomer | | NVP | NVP | NVP | NVP | NVP | NVP | NVP | NVP | NVP |
| | Chain transfer agent | | nDM | nDM | nbDM | nDM | nDM | nDM | nDM | nDM | nDM |
| | Polymerization initiator | | AMBN | AMBN | AMBN | LPO | AMBN | AMBN | AMBN | AMBN | LPO |
| | Solvent | | IPA | IPA | IPA | EtOAc | IPA | IPA | IPA | IPA | EtOAc |
| Reprecipitation step | Poor solvent | Type | DEE/HEX | DIPE/HEX | TBME/HEX | DIPE/HEX | Unpurified | HEX | DIPE | TBME | HEX |
| | | Proportion [% by mass] | 50/50 | 50/50 | 50/50 | 50/50 | - | 100 | 100 | 100 | 100 |
| | Concentration [% by mass] of water soluble polymer solution | | 60 | 60 | 60 | 60 | - | 60 | 60 | 60 | 60 |
| | Amount [parts by mass] of poor solvent | | 1000 | 1000 | 1000 | 1000 | - | 1000 | 1000 | 1000 | 1000 |
| Concentration [ppm by mass] of component (X) | | | 0.1 | 1.0 | 0.9 | 1.2 | 3.3 | 1.4 | 1.8 | 1.7 | 1.5 |
| Evaluation | Appearance | | A | A | A | A | C | B | C | C | B |

Each of abbreviations in Tables 1 and 2 has the following meaning.
· NVP: N-Vinylpyrrolidone.
· nDM: n-Dodecyl mercaptan.
· AMBN: Azobismethylbutyronitrile.
· LPO: Dilauroyl peroxide.
· IPA: Isopropyl alcohol.
· EtOAc: Ethyl acetate.
· DEE: Diethyl ether.
· DIPE: Diisopropyl ether.
· TBME: t-Butyl methyl ether.
· HEX: Hexane.
· TBAH: Tetrabutylammonium hydroxide.

As is clear from Table 1, it was shown that the impurities, that is, the component (X) quantified by GC-FID in the water soluble polymer can be controlled to a predetermined concentration by reprecipitating the water soluble polymer using a poor solvent including an ether or a poor solvent including an ether and an alkane.

In addition, as can be seen from Table 2, it was shown that the foreign matter (defect) of the conductive film could be reduced while maintaining the conductivity and the coating properties, by using the conductive composition including the water soluble composition in which the concentration of impurities was controlled to a predetermined concentration.

## Claims

1. A water soluble composition comprising:
a water soluble polymer,
wherein the water soluble composition satisfies the following Conditions (A1) and (A2),
(A1) the water soluble polymer includes a nitrogen-containing functional group, and
(A2) a 1.0% by mass aqueous solution of the water soluble composition has a surface tension at 25°C of 34.0 to 50.0.

2. The water soluble composition according to Claim 1,
wherein the water soluble composition further satisfies the following Condition (A3),
(A3) the water soluble composition includes more than 0 ppm by mass and 1.3 ppm by mass or less of a component quantified by the following measurement method,
<Measurement Method>
a sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition is dissolved in a mixed solvent of water and methanol at a volume ratio (water/methanol) of 2/8 to give a concentration of 0.2% by mass, such that the volume ratio (solution (I)/solution (II)) is 1/10, and
after injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan.

3. The water soluble composition according to Claim 1,
wherein the water soluble composition further satisfies the following Condition (A4),
(A4) the water soluble composition includes more than 0 ppm by mass and less than 1.0 ppm by mass of a component quantified by the following measurement method,
<Measurement Method>
a sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition is dissolved in a mixed solvent of water and methanol at a volume ratio (water/methanol) of 2/8 to give a concentration of 0.2% by mass, such that the volume ratio (solution (I)/solution (II)) is 1/10, and
after injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan.

4. A water soluble composition comprising:
a water soluble polymer,
wherein the water soluble composition satisfies the following Conditions (B1) to (B3),
(B1) the water soluble polymer includes a nitrogen-containing functional group,
(B2) a 1.0% by mass aqueous solution of the water soluble composition has a surface tension at 25°C of 10.0 to 50.0, and
(B3) the water soluble composition includes more than 0 ppm by mass and 1.3 ppm by mass or less of a component quantified by the following measurement method,
<Measurement Method>
a sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition is dissolved in a mixed solvent of water and methanol at a volume ratio (water/methanol) of 2/8 to give a concentration of 0.2% by mass, such that the volume ratio (solution (I)/solution (II)) is 1/10, and
after injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan.

5. The water soluble composition according to Claim 4,
wherein the water soluble composition further satisfies the following Condition (B4),
(B4) the water soluble composition includes more than 0 ppm by mass and less than 1.0 ppm by mass of a component quantified by the following measurement method,
<Measurement Method>
a sample (S1) is subjected to gas chromatography analysis, the sample (S1) being obtained by mixing a solution (I) in which ethyl n-caproate is dissolved in methanol to give a concentration of 5 mg/L with a solution (II) in which the water soluble composition is dissolved in a mixed solvent of water and methanol at a volume ratio (water/methanol) of 2/8 to give a concentration of 0.2% by mass, such that the volume ratio (solution (I)/solution (II)) is 1/10, and
after injecting the sample (S1) into gas chromatography, in 35-minute analysis in which a time point at which the column oven is heated to 50°C is set as 0 minutes and the temperature is raised to 300°C at 10°C/minute and maintained at 300°C for 10 minutes, a peak area obtained between 25 minutes and 35 minutes is quantified by comparison with a calibration curve created in advance using n-dodecyl mercaptan.

6. The water soluble composition according to Claim 1 or 4,
wherein the water soluble polymer has a mass-average molecular weight of 600 to 20,000.

7. The water soluble composition according to Claim 1 or 4,
wherein the water soluble polymer has a nitrogen-containing functional group.

8. The water soluble composition according to Claim 1 or 4,
wherein the water soluble polymer includes a pyrrolidone structure.

9. A conductive composition comprising:
the water soluble composition according to Claim 1 or 4; and
a conductive polymer.

10. The conductive composition according to Claim 9,
wherein the conductive polymer includes a conductive polymer having at least one unit selected from units represented by General Formulae (1) to (4), 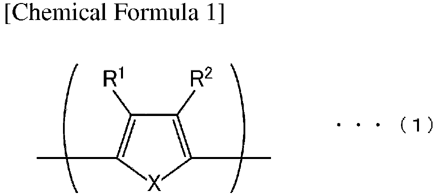 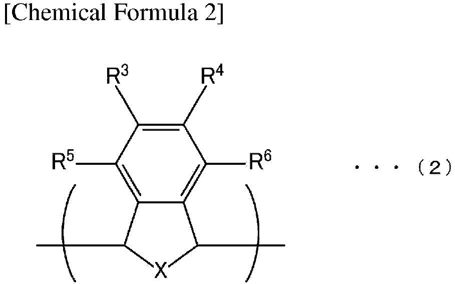 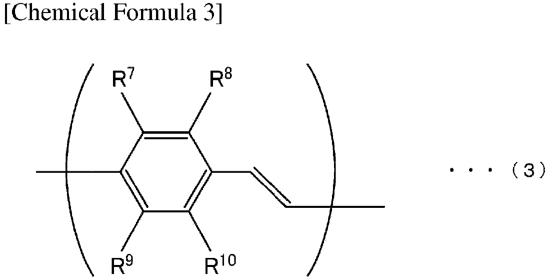 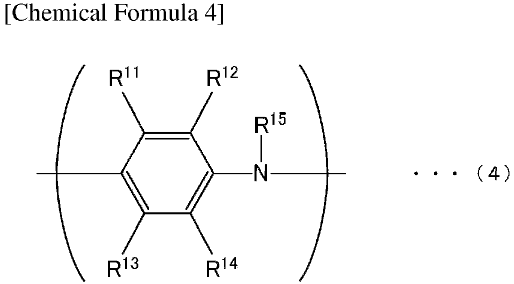
in General Formulae (1) to (4), X represents a sulfur atom or a nitrogen atom, and R¹ to R¹⁵ each independently represent a hydrogen atom, a linear or branched alkyl group having 1 to 24 carbon atoms, a linear or branched alkoxy group having 1 to 24 carbon atoms, an acidic group or a salt of the acidic group, a hydroxy group, a nitro group, a halogen atom, -N(R¹⁶)₂, -NHCOR¹⁶, -SR¹⁶, -OCOR¹⁶, -COOR¹⁶, -COR¹⁶, -CHO, or -CN, and R¹⁶ represents an alkyl group having 1 to 24 carbon atoms, an aryl group having 6 to 24 carbon atoms, or an aralkyl group having 7 to 24 carbon atoms,
provided that at least one of R¹ and R² in General Formula (1), at least one of R³ to R⁶ in General Formula (2), at least one of R⁷ to R¹⁰ in General Formula (3), and at least one of R¹¹ to R¹⁵ in General Formula (4) are each an acidic group or a salt of the acidic group.

11. The conductive composition according to Claim 9,
wherein the conductive polymer includes a conductive polymer having a unit represented by General Formula (4), 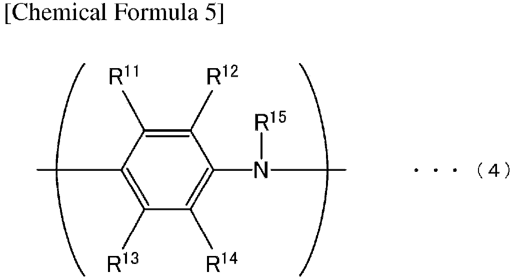
in General Formula (4), R¹¹ to R¹⁵ each independently represent a hydrogen atom, a linear or branched alkyl group having 1 to 24 carbon atoms, a linear or branched alkoxy group having 1 to 24 carbon atoms, an acidic group or a salt of the acidic group, a hydroxy group, a nitro group, a halogen atom, -N(R¹⁶)₂, -NHCOR¹⁶, -SR¹⁶, -OCOR¹⁶, - COOR¹⁶, -COR¹⁶, -CHO, or -CN, and R¹⁶ represents an alkyl group having 1 to 24 carbon atoms, an aryl group having 6 to 24 carbon atoms, or an aralkyl group having 7 to 24 carbon atoms.

12. The conductive composition according to Claim 9, further comprising:
a basic compound.

13. The conductive composition according to Claim 12,
wherein the basic compound includes a quaternary ammonium compound.

14. A method for producing the water soluble composition according to Claim 1 or 4, the method comprising:
a reprecipitation step of mixing a good solvent solution including the water soluble polymer and a good solvent for the water soluble polymer with a poor solvent for the water soluble polymer,
wherein the poor solvent includes an ether.

15. The method for producing the water soluble composition according to Claim 14, the method further comprising:
a synthesis step of the water soluble polymer,
wherein the reprecipitation step is performed after the synthesis step, and
a reaction solution in the synthesis step is used as the good solvent solution in the reprecipitation step.

16. The method for producing the water soluble composition according to Claim 14,
wherein a mixed amount of the poor solvent is 100 parts by mass or more with respect to 100 parts by mass of the good solvent solution.

17. The method for producing the water soluble composition according to Claim 14,
wherein the poor solvent further includes an alkane.

18. The method for producing the water soluble composition according to Claim 17,
wherein a mass ratio of the alkane to the ether (alkane/ether) is 0.5 to 2.0.

19. A method for producing a conductive composition, the method comprising:
a step of producing a water soluble composition by the method for producing the water soluble composition according to Claim 14; and
a step of mixing the water soluble composition obtained in the step of producing the water soluble composition with a conductive polymer.
